# EUROPEAN PATENT SPECIFICATION

(11) **EP 4 395 862 B1**
(45) Date of publication and mention of the grant of the patent: **27.05.2026**
(21) Application number: 22765684.0
(22) Date of filing: 17.08.2022
(51) Int. Cl.: A61M 25/00

(54) **EXPANDABLE SHEATH FOR IMPLANT DELIVERY**
EXPANDIERBARE HÜLLE ZUR IMPLANTATVERABREICHUNG
GAINE EXPANSIBLE POUR POSE D'IMPLANT

(30) Priority: 02.09.2021 US 202163240316 P
(43) Date of publication of application: 10.07.2024
(73) Proprietor: Edwards Lifesciences Corporation, Irvine, CA 92614-5688 (US)
(72) Inventor: PARK, Steven, Irvine, CA 92614 (US); HERNANDEZ, Cristobal R., Irvine, CA 92614 (US)
(74) Representative: Eisenführ Speiser
(86) International application number: PCT/US2022/040638
(87) International publication number: WO 2023/034026

(56) References cited:
- EP-A1- 2 995 268
- US-A1- 2014 012 281
- US-A1- 2018 008 407
- US-A1- 2018 161 064

## Description

### RELATED APPLICATION

This application claims priority to U.S. Provisional Application No. 63/240,316, filed September 2, 2021, and entitled EXPANDABLE SHEATH FOR IMPLANT DELIVERY.

### BACKGROUND

### Field

The present disclosure generally relates to the field of delivering medical implant devices and/or therapies.

### Description of Related Art

Transcatheter delivery of implant devices and/or therapies to the heart can be performed to address various heart abnormalities. Delivery of implant devices and/or therapies to the heart can be performed for treatment of various conditions.

US 2018/161064 A1 describes an introducer which includes an inner liner including a lumen, a proximal region, and at least one folded portion extending longitudinally along the proximal region. The introducer further includes an expandable support member. The support member includes a plurality of ribs extending along a length of the support member. The introducer further includes a sheath attached to at least a portion of the support member and the support member is designed to shift from a first position to an expanded position.

EP 2995268 A1 relates to an expandable introducer sheath for use in inserting a medical device into a body vessel of a patient including a body which extends from a proximal end to a distal end along an axis. The body includes an inner layer, an outer layer, and an expandable reinforcement member which is disposed between said inner and outer layers. The expandable reinforcement member is configured to radially expand as the medical device is axially advanced or retracted through said introducer sheath. Once the medical device has exited the introducer sheath, the expandable reinforcement member facilitates a return of the introducer sheath to its original or unexpanded condition.

US 2014/012281 A1 (US'281) pertains to medical devices and methods for making and using medical devices. An example medical device described in US'281 may include a guide extension catheter. The guide extension catheter may include a proximal member having a proximal outer diameter. A distal sheath member may be attached to the proximal member. The distal sheath member may have a distal outer diameter greater than the proximal outer diameter. The distal sheath member may have a proximal end, a distal end, and a longitudinal slit extending at least partially between the proximal end and the distal end. An expandable member may be attached to the distal sheath member and may extend along the longitudinal slit. The expandable member may be configured to shift between a first configuration and an expanded configuration.

US 2018/008407 A1 concerns an expandable sheath used to deliver a prosthetic device through a patient's vasculature. The sheath is constructed to be expandable in the circumferential direction, while maintaining sufficient stiffness in the longitudinal direction to withstand pushing and resist kinking The sheath includes a plurality of curved arms extending outwardly from a longitudinally extending spine. The curved arms move away from the longitudinal axis of the sheath when pushed radially outwardly by a passing prosthetic device, and move back toward the longitudinal axis once the prosthetic device has passed.

### SUMMARY

For purposes of summarizing the disclosure, certain aspects, advantages, and novel features have been described herein. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

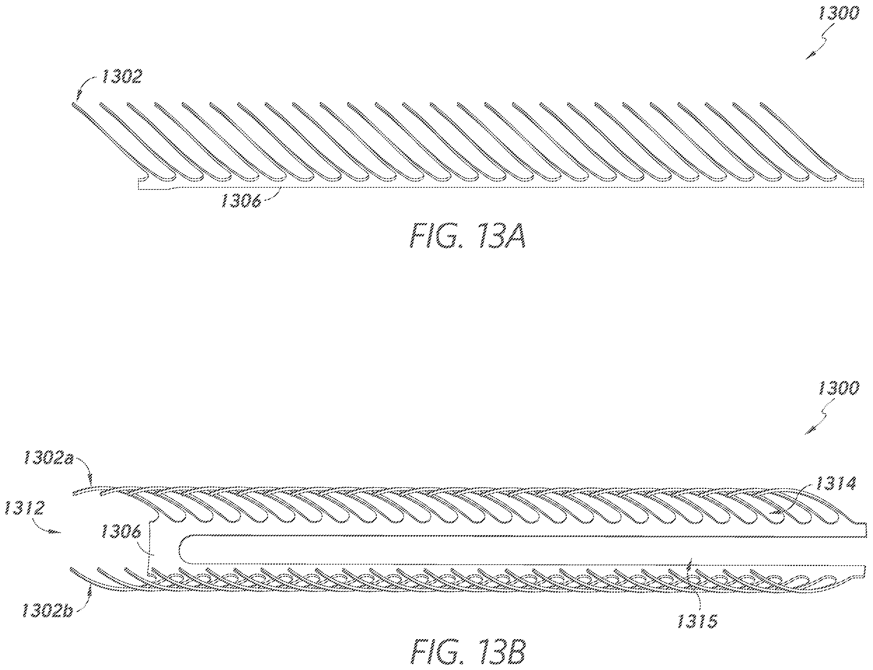

The claimed invention is defined in independent claim 1 and relates to a delivery sheath. Some preferred configurations of the claimed invention are defined in dependent claims 2 to 15. Certain aspects and/or particularly preferred configurations of the claimed invention are discussed hereafter for example in conjunction with Fig. 13B. Also described herein are related aspects, examples, embodiments and arrangements useful for understanding the claimed invention, and which do not necessarily constitute embodiments of the claimed invention. The subject-matter for which protection is sought is defined by the claims.

### BRIEF DESCRIPTION OF THE DRAWINGS

Various examples are depicted in the accompanying drawings for illustrative purposes and should in no way be interpreted as limiting. In addition, various features of different disclosed examples can be combined to form additional examples, which are part of this disclosure. Throughout the drawings, reference numbers may be reused to indicate correspondence between reference elements. However, it should be understood that the use of similar reference numbers in connection with multiple drawings does not necessarily imply similarity between respective examples associated therewith. Furthermore, it should be understood that the features of the respective drawings are not necessarily drawn to scale, and the illustrated sizes thereof are presented for the purpose of illustration of aspects thereof. Generally, certain of the illustrated features may be relatively smaller than as illustrated in some examples or configurations.
Figure 1 illustrates several access pathways for maneuvering guidewires and catheters in and around the heart to deploy compressible medical implants of the present application.
Figure 2 depicts an example method for deploying the medical implants described herein, wherein a guidewire and/or catheter (e.g., sheath) is/are introduced through the subclavian or jugular vein, through the superior vena cava (SVC) and into the coronary sinus.
Figure 3A illustrates a flat-pattern of an expandable sheath configured for delivery of various implants, in accordance with one or more examples.
Figure 3B illustrates the expandable sheath in a recovered/compressed state/form in accordance with one or more examples.
Figure 3C illustrates an expandable sheath in an expanded state in accordance with one or more examples.
Figure 4 illustrates an implant and/or device being inserted through an expandable sheath in accordance with one or more examples.
Figures 5A and 5B illustrate an example expandable sheath comprising offset fingers in accordance with one or more examples.
Figure 6 illustrates an expandable sheath comprising an at least partially expandable coating covering at least a portion of a spine and/or one or more fingers of the expandable sheath in accordance with one or more examples.
Figures 7A and 7B illustrate another example expandable sheath comprising offset fingers having rounded ends in accordance with one or more examples.
Figures 8A and 8B illustrate another example expandable sheath comprising offset fingers in accordance with one or more examples.
Figures 9A and 9B illustrate another example expandable sheath comprising offset fingers having tapered and/or pointed ends in accordance with one or more examples.
Figure 10 illustrates an example expandable sheath comprising in-line fingers in accordance with one or more examples.
Figures 11A and 11B illustrate an example expandable sheath comprising offset and/or twisted fingers in accordance with one or more examples.
Figures 12A and 12B illustrate an example expandable sheath comprising in-line and/or wave-shaped fingers in accordance with one or more examples.
Figures 13A and 13B illustrate an example expandable sheath comprising in-line and/or diagonally-extending fingers in accordance with one or more examples.
Figures 14A-14C illustrate an example expandable sheath comprising helical wires with one or more fingers extending therefrom in accordance with one or more examples.
Figures 15A and 15B illustrate an example expandable sheath comprising in-line and/or wave-shaped fingers extending from spines connected by one or more connecting wires in accordance with one or more examples.
Figures 16A and 16B illustrate an example expandable sheath comprising in-line and/or curved fingers extending from either side of a generally hollow spine in accordance with one or more examples.
Figure 17 illustrates an example expandable sheath comprising one or more helical wires in accordance with one or more examples.

### DETAILED DESCRIPTION

The headings provided herein are for convenience only.

The present disclosure relates to systems, devices, and methods for delivering one or more implants (e.g., medical implants) percutaneously via expandable sheaths. Such implants and/or expandable sheaths may be configured for delivery into and/or through various chambers and/or blood flow pathways of a heart. Some examples herein may describe delivery of one or more implants via an expandable sheath into the coronary sinus from the right atrium via the coronary sinus ostium. However, these examples are for illustrative purposes and the implants and/or expandable sheaths described herein may be configured for delivery into and/or through other areas of a heart.

In any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

### Cardiac Physiology

Heart failure is a common and potentially lethal condition affecting humans, with sub-optimal clinical outcomes often resulting in symptoms, morbidity and/or mortality, despite maximal medical treatment. In particular, "diastolic heart failure" refers to the clinical syndrome of heart failure occurring in the context of preserved left ventricular systolic function (ejection fraction) and in the absence of major valvular disease. This condition is characterized by a stiff left ventricle with decreased compliance and impaired relaxation, which leads to increased end-diastolic pressure. Approximately one third of patients with heart failure have diastolic heart failure and there are very few, if any, proven effective treatments.

Symptoms of diastolic heart failure are due, at least in a large part, to an elevation in pressure in the left atrium. Elevated Left Atrial Pressure (LAP) is present in several abnormal heart conditions, including Heart Failure (HF). In addition to diastolic heart failure, a number of other medical conditions, including systolic dysfunction of the left ventricle and valve disease, can lead to elevated pressures in the left atrium. Both Heart Failure with Preserved Ejection Fraction (HFpEF) and Heart Failure with Reduced Ejection Fraction (HFrEF) can exhibit elevated LAP. It has been hypothesized that both subgroups of HF might benefit from a reduction in LAP, which in turn reduces the systolic preload on the left ventricle, Left Ventricular End Diastolic Pressure (LVEDP). It could also relieve pressure on the pulmonary circulation, reducing the risk of pulmonary edema, improving respiration and improving patient comfort.

The following includes a general description of human cardiac anatomy that is relevant to certain features and examples disclosed herein and is included to provide context for certain aspects of the present disclosure. In humans and other vertebrate animals, the heart is a hollow muscular organ having four pumping chambers: the left and right atria and the left and right ventricles, each provided with its own one-way valve. The natural heart valves are identified as the aortic, mitral (or bicuspid), tricuspid and pulmonary, and are each mounted in an annulus comprising dense fibrous rings attached either directly or indirectly to the atrial and ventricular muscle fibers. Each annulus defines a flow orifice. The four valves ensure that blood does not flow in the wrong direction during the cardiac cycle; that is, to ensure that the blood does not back flow through the valve. Blood flows from the venous system and right atrium through the tricuspid valve to the right ventricle, then from the right ventricle through the pulmonary valve to the pulmonary artery and the lungs. Oxygenated blood then flows through the mitral valve from the left atrium to the left ventricle, and finally from the left ventricle through the aortic valve to the aorta/arterial system.

Heart failure is a common and potentially lethal condition affecting humans, with sub-optimal clinical outcomes often resulting in symptoms, morbidity and/or mortality, despite maximal medical treatment. In particular, "diastolic heart failure" refers to the clinical syndrome of heart failure occurring in the context of preserved left ventricular systolic function (ejection fraction) and in the absence of major valvular disease. This condition is characterized by a stiff left ventricle with decreased compliance and impaired relaxation, which leads to increased end-diastolic pressure. Approximately one third of patients with heart failure have diastolic heart failure and there are very few, if any, proven effective treatments.

Pulmonary hypertension (PH) is defined as a rise in mean pressure in the main pulmonary artery. PH may arise from many different causes, but, in all patients, has been shown to increase mortality rate. A deadly form of PH arises in the very small branches of the pulmonary arteries and is known as Pulmonary Arterial Hypertension (PAH). In PAH, the cells inside the small arteries multiply due to injury or disease, decreasing the area inside of the artery and thickening the arterial wall. As a result, these small pulmonary arteries narrow and stiffen, causing blood flow to become restricted and upstream pressures to rise. This increase in pressure in the main pulmonary artery is the common connection between all forms of PH regardless of underlying cause. Despite previous attempts, there is a need for an improved way to reduce elevated pressure in the left atrium, as well as other susceptible heart chambers such as the pulmonary artery.

The present disclosure provides methods and devices for delivering various implants to desired locations within a human body. The term "implant" is used herein according to its plain and ordinary meaning and may refer to any medical implant, frame, valve, shunt, stent, anchor, and/or similar devices for use in treating various conditions in a human body. Implants may be delivered via one or more expandable sheaths and/or catheters configured for various medical procedures. The terms "sheath" and/or "catheter" are used herein according to their broad and ordinary meaning and may include any tubes, sheaths, steerable sheaths, steerable catheters, and/or any other type of elongate tubular delivery device comprising an inner lumen configured to slidably receive one or more implants. Implants described herein may be configured to be shaped and/or compressed to fit into a catheter.

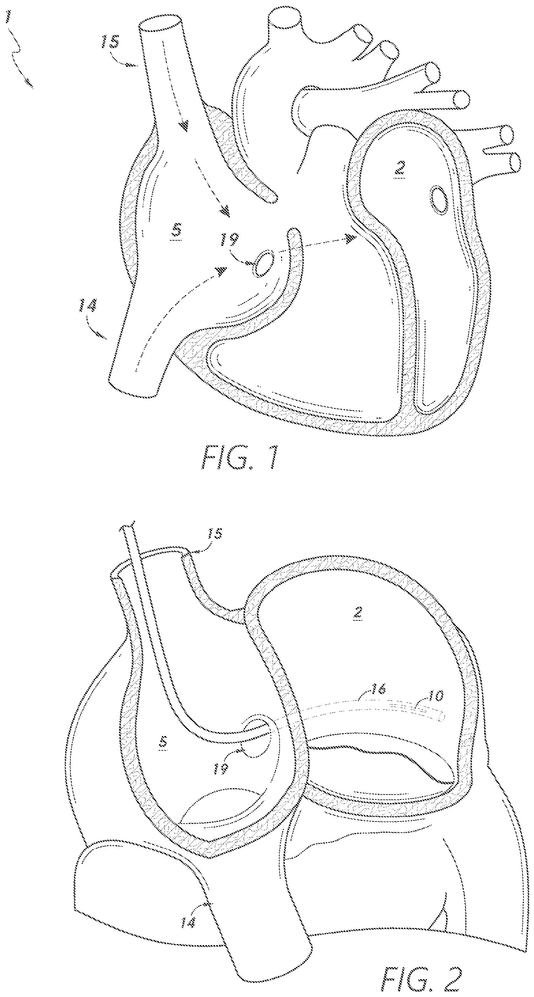

Figure 1 illustrates several access pathways for maneuvering guidewires and/or expandable catheters in and around the heart 1 to deploy medical implants of the present application. For instance, access may be from above via either the subclavian vein or jugular vein into the superior vena cava (SVC) 15, right atrium (RA) 5 and from there into the coronary sinus (CS) 19. Alternatively, the access path may start in the femoral vein and through the inferior vena cava (IVC) 14 into the heart 1. Other access routes may also be used, and each typically utilizes a percutaneous incision through which the guidewire and catheter are inserted into the vasculature, normally through a sealed introducer, and from there the physician controls the distal ends of the devices from outside the body.

Figure 2 depicts an example method for deploying medical implants 10, wherein a guidewire and/or catheter 16 (e.g., sheath) is/are introduced through the subclavian or jugular vein, through the SVC 15 and into the coronary sinus 19. However, implants may be deployed using different means and/or different delivery paths. In some instances, a guidewire may be used to provide a path, after which an introducer sheath (not shown) may be routed along the guidewire and into the patient's vasculature, typically with the use of a dilator. Figure 2 shows a deployment catheter 16 extending from the SVC 15 to the coronary sinus 19 of the heart 1, the deployment catheter 16 having been passed through the introducer sheath which provides a hemostatic valve to prevent blood loss.

In one example, the deployment catheter 16 may be about 30 cm long, and the guidewire may be somewhat longer for ease of use. In some examples, the deployment catheter may function to form and prepare an opening in the wall of the left atrium 2, and a separate expandable placement and/or delivery catheter will be used for delivery of an implant 10. In other examples, the deployment catheter may be used as both the puncture preparation and implant placement catheter with full functionality.

While implants 10 for use in treating LAP and/or PH have been described, this is for illustrative purposes only and the delivery devices and/or methods described herein may be configured for different uses and/or procedures. Moreover, while delivery via the coronary sinus has been described, this is for illustrative purposes only and the sheaths described herein may be configured for delivery via any blood flow pathway and/or chamber of the heart.

### Expandable Sheaths

Examples of the present disclosure relate to delivery of various implants via one or more expandable sheaths configured to expand and/or compress in response to movement of the implants through inner lumens of the expandable sheaths. In some examples, an expandable sheath may be configured for delivery at least partially through a blood flow pathway (e.g., a blood vessel). Some expandable sheaths described herein can be configured to reduce vascular complications by reducing trauma when inserting an implant through the expandable sheath. The expandable sheath can reduce in size after the device is delivered through the expandable sheath, thereby minimizing interaction/contact between the expandable sheath and a blood flow pathway the expandable sheath and/or implant may be passed through.

In some examples, an expandable sheath can be laser-cut and/or may be composed of one or more shape-memory materials and/or alloys (e.g., Nitinol). For example, an expandable sheath may be shape-set to a compressed form. In some examples, an expandable sheath may have a generally flexible and/or elastic structure. For example, when an implant is passed through a sheath, at least a portion of the sheath may be configured to expand around the implant and/or to re-assume a compressed form after the implant passes through at least the portion of the sheath.

Expandable sheaths described herein may have variable forms. For example, a tube (e.g., a Nitinol tube) may be laser-cut in various patterns to improve expandability of the tube. In some examples, an expandable sheath may have a noncontinuous surface and/or may comprise a network of gaps and/or cells formed in a frame of the sheath. For example, the expandable sheath may comprise a network of wires and/or struts around cells and/or gaps. In some examples, an expandable sheath may be at least partially enclosed by a coating and/or covering, which may at least partially cover at least some cells and/or gaps of the sheath.

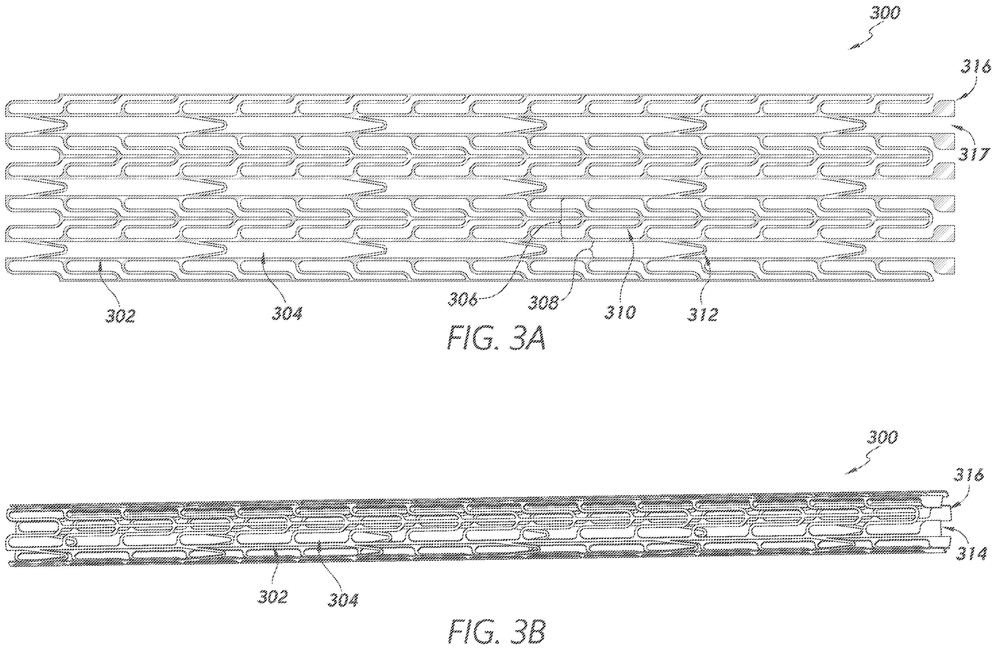

Figure 3A illustrates a flat-pattern of an expandable sheath 300 configured for delivery of various implants, in accordance with one or more examples. The expandable sheath 300 can comprise a network of inter-connected struts 302 (i.e., fingers) and/or wires extending around gaps 304 and/or cavities. The terms "strut" and "finger" are used herein to refer to any generally thin, bendable, and/or extendible length of material configured to at least partially enclose an inner lumen of an expandable sheath. A strut 302 and/or finger can comprise any means for at least partially enclosing an inner lumen 314 and/or means for expanding the inner lumen 314 and/or can include one or more wires, cords, bars, and/or similar devices and/or may be interconnected with various other struts 302 and/or fingers and/or may form rib-like extensions from one or more wires and/or base portions (e.g., spines). The term "spine" is used herein in accordance with its plain and ordinary meaning and may refer to any length of one or more materials (which can include shape-memory alloys, such as Nitinol) configured to provide a base for one or more rib-like and/or finger-like extensions (e.g., cords, wires, bars) extending from the spine. A spine may extend along a length of an expandable sheath 300 and/or an inner lumen 314 through the sheath 300 and/or may include any means for enclosing an inner lumen 314. In some examples, the expandable sheath 300 may be formed from a generally flat and/or tubular device composed at least partially of a generally elastic and/or flexible material. For example, the expandable sheath 300 may be formed by laser-cutting and/or otherwise cutting a generally tubular sheet of material formed at least partially of one or more shape-memory alloys (e.g. Nitinol). The gaps 304 may represent portions of the sheet of material removed during the cutting process.

The network of struts 302 may have any of a variety of forms and/or may comprise distinct sections having different forms. For example, the expandable sheath 300 may comprise a first pattern 306 of struts 302 and/or may comprise a second pattern 308 of struts 302. Patterns of struts 302 may extend and/or repeat longitudinally (i.e., lengthwise) along the sheath 300 and/or may change laterally along the sheath 300. In some examples, portions of the expandable sheath 300 having the first pattern 306 may be situated between two portions of the expandable sheath 300 having the second pattern 308. Similarly, portions of the expandable sheath 300 having the second pattern 308 may be situated between two portions of the expandable sheath 300 having the first pattern 306. The first pattern 306 may comprise W- and/or trident-shaped struts 310, which may include a generally straight strut between two curved struts. The second pattern 308 may comprise V- and/or U-shaped struts 312. Portions of the expandable sheath 300 having the first pattern 306 may be separated by portions of the expandable sheath 300 having the second pattern 308 by generally straight struts 302 extending longitudinally along the sheath 300.

The expandable sheath 300 may comprise one or more end portions 316 configured to form a circular and/or partial-circular opening into an inner lumen 314 of the expandable sheath 300 when the expandable sheath 300 is formed into a cylindrical and/or tubular form. In some examples, the end portions 316 together form a full circular shape. In other examples, one or more gaps 317 may separate the end portions 316.

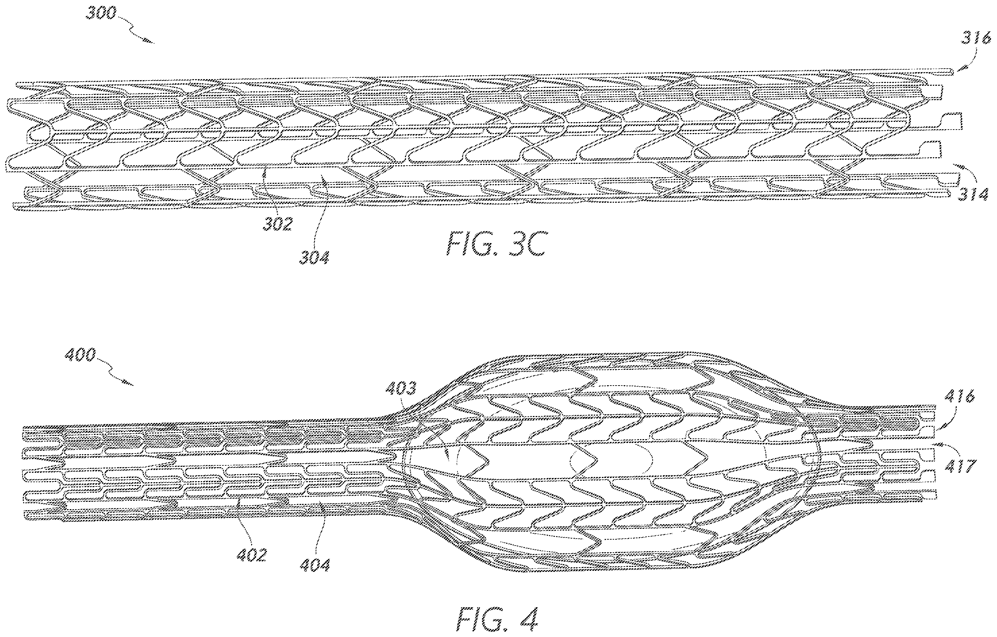

Figure 3B illustrates the generally tubular expandable sheath 300 in a recovered/compressed state/form, in which the expandable sheath 300 has a reduced diameter/width in comparison to an expanded state (shown in Figure 3C). The compressed state can be the resting state of the expandable sheath 300. For example, the expandable sheath 300, including the network of struts 302, may be shape-set in the compressed form. The expandable sheath 300 may be inserted into a vasculature in the compressed state and/or may be configured to return to the compressed state after one or more implants pass through the expandable sheath 300. The sheath 300 can comprise a laser-cut Nitinol tube that can be shape-set in the compressed form and/or may be coated in an at least partially expandable material.

Figure 3C illustrates the expandable sheath 300 in an expanded state, in which the expandable sheath 300 has an increased diameter and/or width in comparison to the compressed state shown in Figure 3B. The compressed state can be the working and/or shape-set state/form of the expandable sheath 300. When a device (e.g., a medical implant) is inserted or retrieved through the inner lumen 314 of the expandable sheath 300, at least a portion of the expandable sheath 300 can expand in one or more radial directions to allow the at least a portion of the expandable sheath 300 to accommodate the device. **In** some examples, one or more struts 302 of the expandable sheath 300 may be configured to at least partially bend in response to the device being inserted through the inner lumen 314 of the expandable sheath 300. For example, the curved struts 302 forming the first pattern 306 and/or second pattern 308 of the sheath 300 may be configured to straighten and/or extend laterally to increase the width and/or diameter of the inner lumen 314 of the sheath 300. The struts 302 may form an at least partial cylinder around the inner lumen 314.

Figure 4 illustrates an implant 403 and/or device being inserted through an expandable sheath 400. The sheath 400 may be configured to naturally expand in response to the implant 403 being inserted through the sheath 400. The sheath 400 may comprise a network of struts 402 (i.e., fingers) forming and/or extending around one or more gaps 404. The sheath 400, in a compressed and/or default form, may have a width and/or diameter that is less than a width and/or diameter of the implant 403. Accordingly, as the implant 403 is extended through an inner lumen of the expandable sheath 400, the implant 403 may cause expansion of the struts 402 of the sheath 400. In some examples, the sheath 400 may be configured to expand around the implant 403 and/or to elastically return to a compressed and/or default form after the implant 403 passes at least partially through the sheath 400.

As shown in Figure 4, as the implant 403 passes through the inner lumen of the sheath 400, the struts 402 situated around the implant 403 may be configured to bend, straighten, and/or extend laterally to accommodate the implant 403 while other struts 402 not situated around the implant 403 may maintain a default compressed form. As the implant 403 moves through the inner lumen of the sheath 400, different struts 402 may extend while previously extended struts 402 may compress.

The expandable sheath 400 may comprise one or more end portions 416 configured to form a circular and/or partial-circular opening into an inner lumen of the expandable sheath 400 when the expandable sheath 400 is formed into a cylindrical form. In some examples, the end portions 416 form a full circular shape. In other examples, one or more gaps 417 may separate the end portions 416.

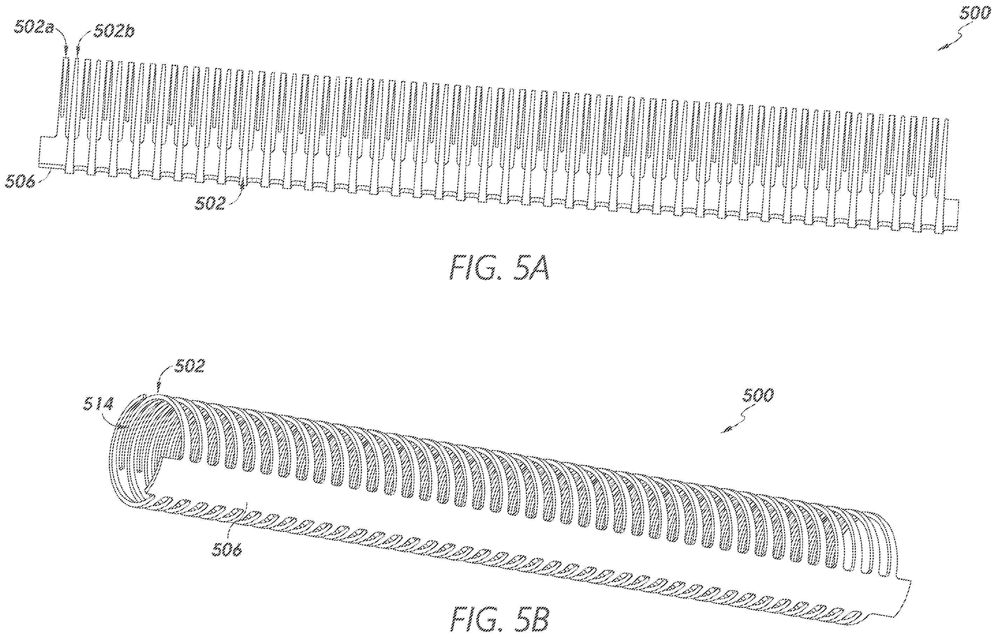

Figures 5A and 5B illustrate an example expandable sheath 500 comprising offset fingers 502 in accordance with one or more examples. The expandable sheath 500 may have a generally tubular and/or cylindrical shape and/or may comprise one or more fingers 502 (i.e., extensions, teeth, and/or elongate members) extending around an inner lumen 514 of the expandable sheath 500. The one or more fingers 502 and/or inner lumen 514 may form a generally circular cross section. The expandable sheath 500 may comprise a spine 506 extending along at least a portion of the expandable sheath 500. In some examples, the one or more fingers 502 may be inter-connected via connections between the one or more fingers 502 and the spine 506. However, the one or more fingers 502 may not directly couple to and/or contact each other. For example, the one or more fingers 502 may comprise free ends to allow the one or more fingers 502 to bend and/or extend as needed to accommodate one or more implants. The spine 506 may have a generally solid (e.g., having no apertures and/or gaps), curved (e.g., forming a portion of a circular cross-section of the sheath 500), and/or rectangular form and/or may comprise a sheet of any suitable material. The one or more fingers 502 and/or spine 506 may form an at least partial cylinder around the inner lumen 514. In some examples, the spine 506 may extend along a first axis and/or plane and/or at least some of the one or more fingers 502 may extend generally perpendicularly form the first axis and/or along a second axis and/or plane (and/or additional axes and/or planes) that is/are orthogonal, perpendicular, and/or at an angle from the first axis and/or plane.

In some examples, the one or more fingers 502 may have a generally curved form and/or may be shape-set in a semi-circular and/or partial-circular shape. The one or more fingers 502 may be configured to extend from either side and/or two sides of the spine 506. In some examples, one or more fingers 502 extending from a first side of the spine 506 may be in-line and/or offset from one or more fingers 502 extending from a second side of the spine 506. For example, a first finger 502a may be at least partially offset from a second finger 502b. The first finger 502a may extend from a first side of the spine 506 and the second finger 502b may extend from a second side of the spine 506. In some examples, the first finger 502a may be configured to extend at least partially along the second finger 502b in a compressed and/or expanded form of the expandable sheath 500 and/or the first finger 502a may not be in direct contact with the second finger 502b. For example, the first finger 502a and the second finger 502b may at least partially overlap with or without contacting each other. Other fingers 502 may similarly overlap with each other. In some examples, as the expandable sheath 500 expands, an amount of overlap between the one or more fingers 502 may decrease as the fingers 502 straighten and/or are pressed away from each other by an implant passing through the inner lumen 514.

The expandable sheath 500 is shown in a compressed or expanded form. In some examples, the one or more fingers 502 may be configured to overlap in a compressed form and/or not to overlap in an expanded form. Expansion of the expandable sheath 500 may cause one or more fingers 502 to at least somewhat straighten and/or extend laterally (i.e., perpendicularly to the length of the sheath 500).

The spine 506 may have any suitable size and/or shape. In some examples, the spine 506 may have a generally rectangular shape and/or may be at least partially curved to facilitate a curvature and/or tubular shape of the expandable sheath 500. While the spine 506 is shown comprising a sheet of material, the spine 506 may be at least partially porous and/or may comprise one or more apertures and/or openings.

The one or more fingers 502 may have any suitable size and/or shape. In some examples, at least some of the one or more fingers 502 may have a thin and/or wire-like form. The one or more fingers 502 may be configured to bend in response to implants passing through the inner lumen 514. In some examples, the one or more fingers 502 may be at least partially composed of one or more shape-memory alloys (e.g., Nitinol) and/or may be configured to elastically assume the form shown in Figure 5A and/or 5B upon removal of an implant and/or other external force.

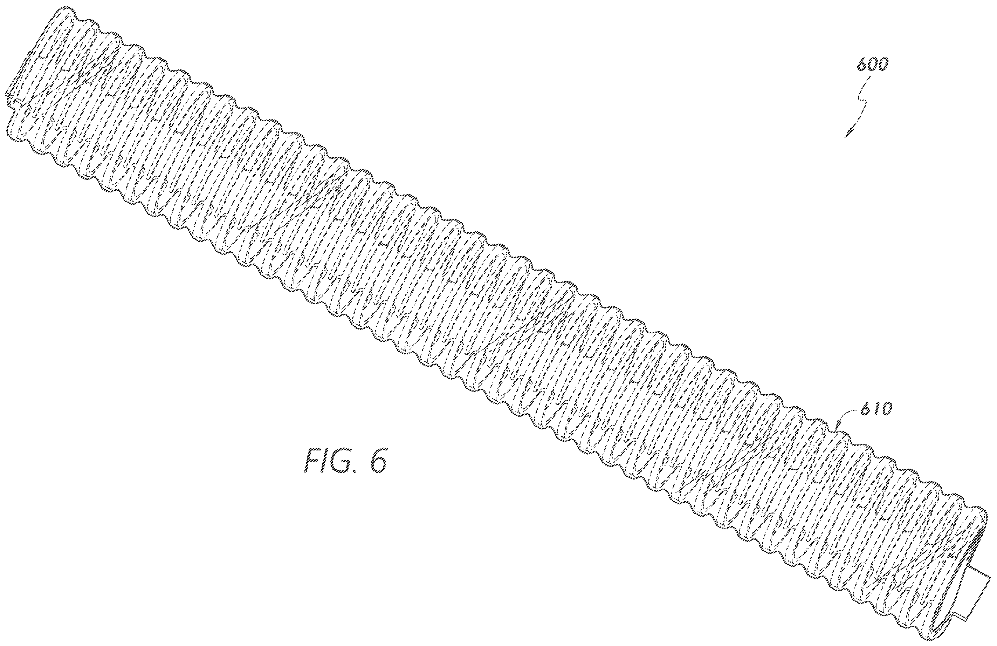

Figure 6 illustrates an expandable sheath 600 comprising an at least partially expandable coating 610 covering at least a portion of a spine and/or one or more fingers of the expandable sheath in accordance with one or more examples. An expandable coating 610 may be applied to any of the various expandable sheaths 600 described and/or illustrated herein.

In some examples, the expandable coating 610 may be applied to one or more spines and/or fingers of an expandable sheath 600 via a dipping process. An outer surface of an expandable sheath 600 (e.g., an outer surface of one or more fingers and/or a spine) may be primed and/or etched to prepare the expandable sheath 600 for coating. The outer surface may be coated in any of a variety of suitable compliant materials, which can include latex, silicone, thermoplastic Polyurethane (TPU), and/or expanded Polytetrafluoroethylene (ePTFE). The coating may be configured to expand with expansion of the fingers and/or other portions of the expandable sheath 600. In some examples, the expandable coating 610 may be configured to cover and/or fill any gaps, apertures, and/or openings of the expandable sheath 600, which can include spaces between fingers, apertures in a spine, etc. The expandable coating 610 may be configured to prevent damage to tissue from snagging which may be caused by fingers and/or spines of the expandable sheath 600 when the expandable sheath 600 is delivered through tissue pathways. In some examples, the expandable coating 610 may be configured to form a generally smooth surface.

The expandable coating 610 may further be coated by one or more additional materials. For example, one or more hydrophilic materials may be applied to the expandable coating 610 to facilitate delivery of the expandable sheath 600 through one or more blood flow pathways. In some examples, the expandable coating 610 and/or additional coatings may be configured to cover an inner surface (e.g., an inner lumen-facing surface) of the expandable sheath to prevent snagging as one or more implants are passed through the inner lumen of the expandable sheath 600.

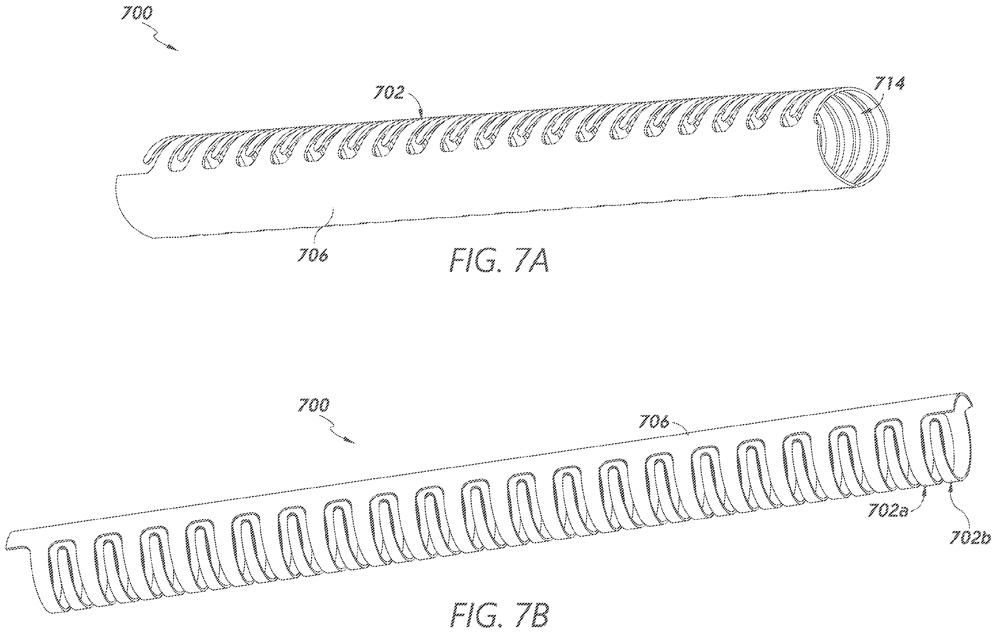

Figures 7A and 7B illustrate another example expandable sheath 700 comprising offset fingers 702 having rounded ends in accordance with one or more examples. The expandable sheath 700 may have a generally tubular and/or cylindrical shape and/or may comprise one or more fingers 702 (i.e., extensions, teeth, and/or elongate members) extending around an inner lumen 714 of the expandable sheath 700. The one or more fingers 702 and/or inner lumen 714 may form a generally circular cross section. The expandable sheath 700 may comprise a spine 706 extending along at least a portion of the expandable sheath 700. In some examples, the one or more fingers 702 may be inter-connected via connections between the one or more fingers 702 and the spine 706. However, the one or more fingers 702 may not directly couple to and/or contact each other. For example, the one or more fingers 702 may comprise free ends to allow the one or more fingers 702 to bend and/or extend as needed to accommodate one or more implants. The one or more fingers 702 and/or spine 706 may form an at least partial cylinder around the inner lumen 714.

In some examples, the one or more fingers 702 may have a generally curved form and/or may be shape-set in a semi-circular and/or partial-circular shape. The one or more fingers 702 may be configured to extend from either side and/or two sides of the spine 706. In some examples, one or more fingers 702 extending from a first side of the spine 706 may be in-line and/or offset from one or more fingers 702 extending from a second side of the spine 706. For example, a first finger 702a may be at least partially offset from a second finger 702b. The first finger 702a may extend from a first side of the spine 706 and the second finger 702b may extend from a second side of the spine 706. In some examples, the first finger 702a may be configured to extend at least partially along the second finger 702b in a compressed and/or expanded form of the expandable sheath 700. For example, the first finger 702a and the second finger 702b may at least partially overlap with or without contacting each other. Other fingers 702 may similarly overlap with each other. In some examples, as the expandable sheath 700 expands, an amount of overlap between the one or more fingers 702 may decrease as the fingers 702 straighten and/or are pressed away from each other by an implant passing through the inner lumen 714.

The expandable sheath 700 is shown in a compressed or expanded form. In some examples, the one or more fingers 702 may be configured to overlap in a compressed form and/or not to overlap in an expanded form. Expansion of the expandable sheath 700 may cause one or more fingers 702 to at least somewhat straighten.

The spine 706 may have any suitable size and/or shape. In some examples, the spine 706 may have a generally rectangular shape and/or may be at least partially curved to facilitate a curvature and/or tubular shape of the expandable sheath 700. While the spine 706 is shown comprising a sheet of material, the spine 706 may be at least partially porous and/or may comprise one or more apertures and/or openings.

The one or more fingers 702 may have any suitable size and/or shape. In some examples, at least some of the one or more fingers 702 may have a flat and/or rounded form. The one or more fingers 702 may be configured to bend in response to implants passing through the inner lumen 714. In some examples, the one or more fingers 702 may be at least partially composed of one or more shape-memory alloys (e.g., Nitinol) and/or may be configured to elastically assume the form shown in Figure 7A and/or 7B upon removal of an implant and/or other external force.

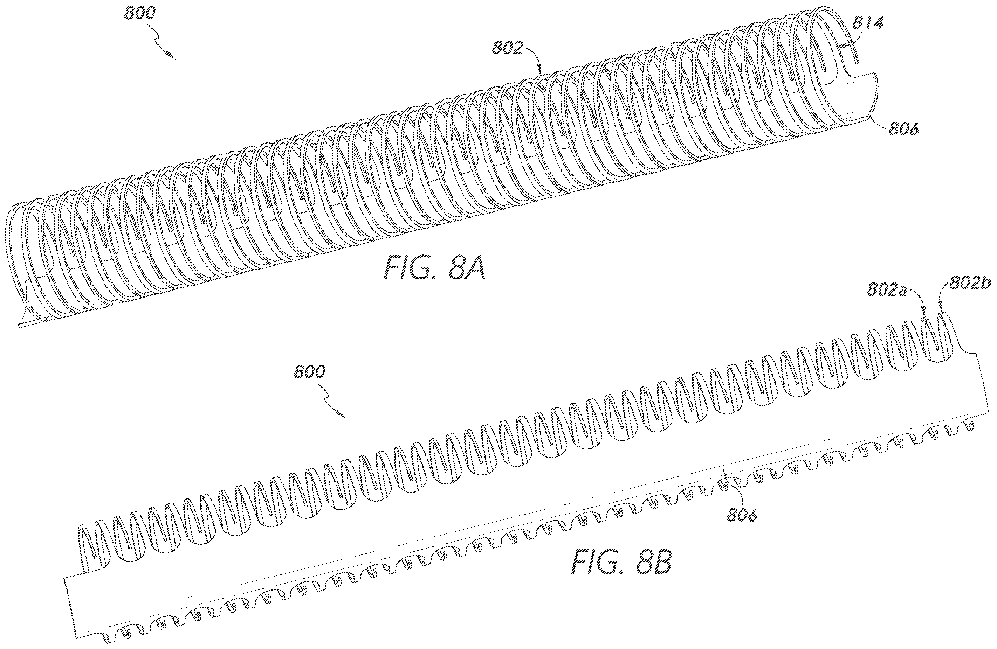

Figures 8A and 8B illustrate another example expandable sheath 800 comprising offset fingers 802 in accordance with one or more examples. The expandable sheath 800 may have a generally tubular and/or cylindrical shape and/or may comprise one or more fingers 802 (i.e., extensions, teeth, and/or elongate members) extending around an inner lumen 814 of the expandable sheath 800. The one or more fingers 802 and/or inner lumen 814 may form a generally circular cross section. The expandable sheath 800 may comprise a spine 806 extending along at least a portion of the expandable sheath 800. In some examples, the one or more fingers 802 may be inter-connected via connections between the one or more fingers 802 and the spine 806. However, the one or more fingers 802 may not directly couple to and/or contact each other. For example, the one or more fingers 802 may comprise free ends to allow the one or more fingers 802 to bend and/or extend as needed to accommodate one or more implants. The one or more fingers 802 and/or spine 806 may form an at least partial cylinder around the inner lumen 814.

In some examples, the one or more fingers 802 may have a generally curved form and/or may be shape-set in a semi-circular and/or partial-circular shape. The one or more fingers 802 may be configured to extend from either side and/or two sides of the spine 806. In some examples, one or more fingers 802 extending from a first side of the spine 806 may be in-line and/or offset from one or more fingers 802 extending from a second side of the spine 806. For example, a first finger 802a may be at least partially offset from a second finger 802b. The first finger 802a may extend from a first side of the spine 806 and the second finger 802b may extend from a second side of the spine 806. In some examples, the first finger 802a may be configured to extend at least partially along the second finger 802b in a compressed and/or expanded form of the expandable sheath 800. For example, the first finger 802a and the second finger 802b may at least partially overlap with or without contacting each other. Other fingers 802 may similarly overlap with each other. In some examples, as the expandable sheath 800 expands, an amount of overlap between the one or more fingers 802 may decrease as the fingers 802 straighten and/or are pressed away from each other by an implant passing through the inner lumen 814.

The expandable sheath 800 is shown in a compressed or expanded form. In some examples, the one or more fingers 802 may be configured to overlap in a compressed form and/or not to overlap in an expanded form. Expansion of the expandable sheath 800 may cause one or more fingers 802 to at least somewhat straighten.

The spine 806 may have any suitable size and/or shape. In some examples, the spine 806 may have a generally rectangular shape and/or may be at least partially curved to facilitate a curvature and/or tubular shape of the expandable sheath 800. While the spine 806 is shown comprising a sheet of material, the spine 806 may be at least partially porous and/or may comprise one or more apertures and/or openings.

The one or more fingers 802 may have any suitable size and/or shape. In some examples, at least some of the one or more fingers 802 may have a thin and/or wire-like form. The one or more fingers 802 may be configured to bend in response to implants passing through the inner lumen 814. In some examples, the one or more fingers 802 may be at least partially composed of one or more shape-memory alloys (e.g., Nitinol) and/or may be configured to elastically assume the form shown in Figure 8A and/or 8B upon removal of an implant and/or other external force.

The spine 806 may have a variable width. In some examples, sides of the spine 806 may have an uneven, weaving, and/or wave-like form in which the sides of the spine 806 extend out into the one or more fingers 802. For example, the spine 806 may form one or more peaks and/or troughs and/or the one or more fingers 802 may extend from peaks of the spine 806.

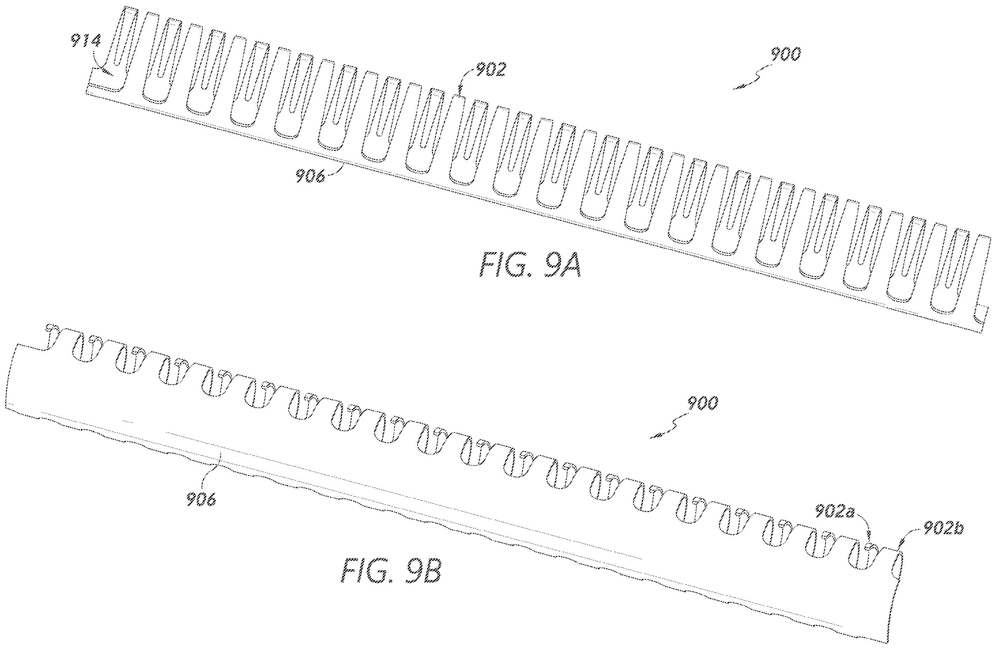

Figures 9A and 9B illustrate another example expandable sheath 900 comprising offset fingers 902 having tapered and/or pointed ends in accordance with one or more examples. The expandable sheath 900 may have a generally tubular and/or cylindrical shape and/or may comprise one or more fingers 902 (i.e., extensions, teeth, and/or elongate members) extending around an inner lumen 914 of the expandable sheath 900. The one or more fingers 902 and/or inner lumen 914 may form a generally circular cross section. The expandable sheath 900 may comprise a spine 906 extending along at least a portion of the expandable sheath 900. In some examples, the one or more fingers 902 may be inter-connected via connections between the one or more fingers 902 and the spine 906. However, the one or more fingers 902 may not directly couple to and/or contact each other. For example, the one or more fingers 902 may comprise free ends to allow the one or more fingers 902 to bend and/or extend as needed to accommodate one or more implants.

In some examples, the one or more fingers 902 may have a generally curved form and/or may be shape-set in a semi-circular and/or partial-circular shape. The one or more fingers 902 may be configured to extend from either side and/or two sides of the spine 906. In some examples, one or more fingers 902 extending from a first side of the spine 906 may be in-line and/or offset from one or more fingers 902 extending from a second side of the spine 906. For example, a first finger 902a may be at least partially offset from a second finger 902b. The first finger 902a may extend from a first side of the spine 906 and the second finger 902b may extend from a second side of the spine 906. In some examples, the first finger 902a may be configured to extend at least partially along the second finger 902b in a compressed and/or expanded form of the expandable sheath 900. For example, the first finger 902a and the second finger 902b may at least partially overlap with or without contacting each other. Other fingers 902 may similarly overlap with each other. In some examples, as the expandable sheath 900 expands, an amount of overlap between the one or more fingers 902 may decrease as the fingers 902 straighten and/or are pressed away from each other by an implant passing through the inner lumen 914. The one or more fingers 902 and/or spine 906 may form an at least partial cylinder around the inner lumen 914.

The expandable sheath 900 is shown in a compressed or expanded form. In some examples, the one or more fingers 902 may be configured to overlap in a compressed form and/or not to overlap in an expanded form. Expansion of the expandable sheath 900 may cause one or more fingers 902 to at least somewhat straighten.

The spine 906 may have any suitable size and/or shape. In some examples, the spine 906 may have a generally rectangular shape and/or may be at least partially curved to facilitate a curvature and/or tubular shape of the expandable sheath 900. While the spine 906 is shown comprising a sheet of material, the spine 906 may be at least partially porous and/or may comprise one or more apertures and/or openings.

The one or more fingers 902 may have any suitable size and/or shape. In some examples, at least some of the one or more fingers 902 may have a thin and/or wire-like form. The one or more fingers 902 may be configured to bend in response to implants passing through the inner lumen 914. In some examples, the one or more fingers 902 may be at least partially composed of one or more shape-memory alloys (e.g., Nitinol) and/or may be configured to elastically assume the form shown in Figure 9A and/or 9B upon removal of an implant and/or other external force.

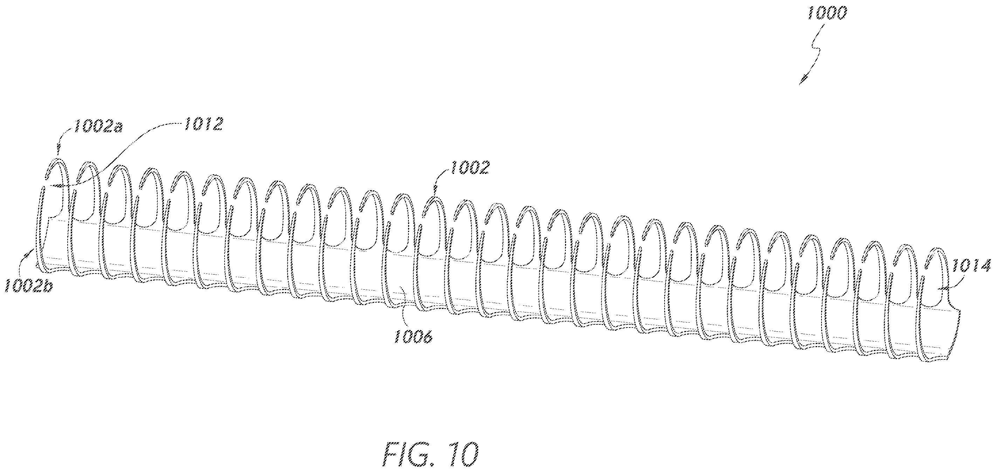

Figure 10 illustrates an example expandable sheath 1000 comprising in-line fingers 1002 in accordance with one or more examples. The expandable sheath 1000 may have a generally tubular and/or cylindrical shape and/or may comprise one or more fingers 1002 (i.e., extensions, teeth, and/or elongate members) extending around an inner lumen 1014 of the expandable sheath 1000. The one or more fingers 1002 and/or inner lumen 1014 may form a generally circular cross section. The expandable sheath 1000 may comprise a spine 1006 extending along at least a portion of the expandable sheath 1000. In some examples, the one or more fingers 1002 may be inter-connected via connections between the one or more fingers 1002 and the spine 1006. However, the one or more fingers 1002 may not directly couple to and/or contact each other. For example, the one or more fingers 1002 may comprise free ends to allow the one or more fingers 1002 to bend and/or extend as needed to accommodate one or more implants. The one or more fingers 1002 and/or spine 1006 may form an at least partial cylinder around the inner lumen 1014.

In some examples, the one or more fingers 1002 may have a generally curved form and/or may be shape-set in a semi-circular and/or partial-circular shape. The one or more fingers 1002 may be configured to extend from either side and/or two sides of the spine 1006. In some examples, one or more fingers 1002 extending from a first side of the spine 1006 may be in-line and/or offset from one or more fingers 1002 extending from a second side of the spine 1006. For example, a first finger 1002a may be at least partially in-line with a second finger 1002b. The first finger 1002a may extend from a first side of the spine 1006 and the second finger 1002b may extend from a second side of the spine 1006. In some examples, there may be a gap 1012 between an end of the first finger 1002a and an end of the second finger 1002b. In some examples, as the expandable sheath 1000 expands, the gap 1012 between the fingers 1002 may increase as the fingers 1002 straighten and/or are pressed away from each other by an implant passing through the inner lumen 1014.

The expandable sheath 1000 is shown in a compressed or expanded form. In some examples, the one or more fingers 1002 may be configured to overlap in a compressed form and/or not to overlap in an expanded form. Expansion of the expandable sheath 1000 may cause one or more fingers 1002 to at least somewhat straighten.

The spine 1006 may have any suitable size and/or shape. In some examples, the spine 1006 may have a generally rectangular shape and/or may be at least partially curved to facilitate a curvature and/or tubular shape of the expandable sheath 1000. While the spine 1006 is shown comprising a sheet of material, the spine 1006 may be at least partially porous and/or may comprise one or more apertures and/or openings.

The one or more fingers 1002 may have any suitable size and/or shape. In some examples, at least some of the one or more fingers 1002 may have a thin and/or wire-like form. The one or more fingers 1002 may be configured to bend in response to implants passing through the inner lumen 1014. In some examples, the one or more fingers 1002 may be at least partially composed of one or more shape-memory alloys (e.g., Nitinol) and/or may be configured to elastically assume the form shown in Figure 10 upon removal of an implant and/or other external force.

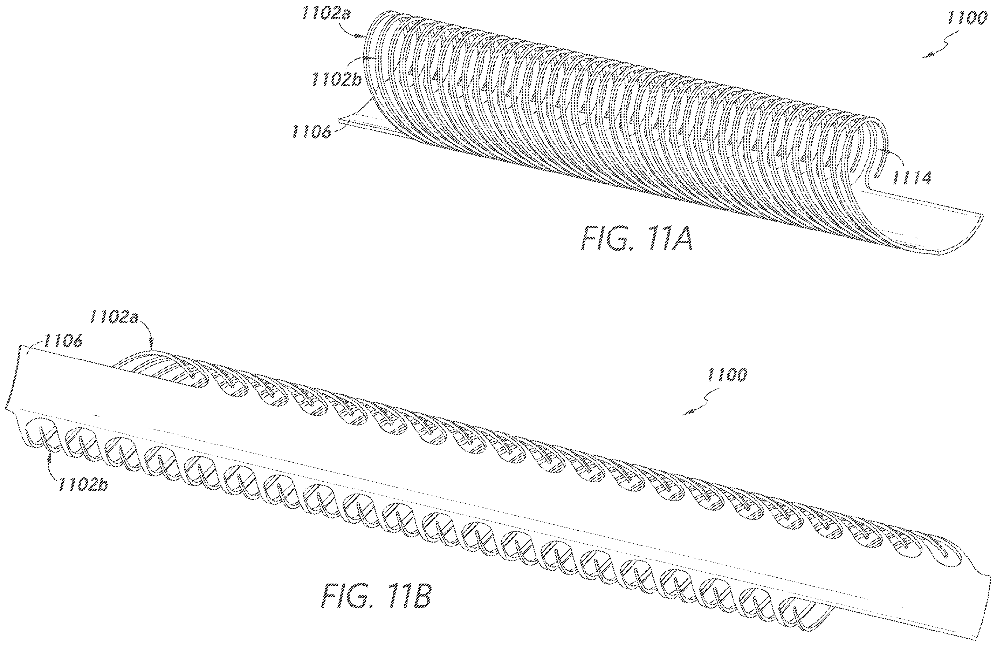

Figures 11A and 11B illustrate an example expandable sheath 1100 comprising offset and/or twisted fingers 1102 in accordance with one or more examples. The expandable sheath 1100 may have a generally tubular and/or cylindrical shape and/or may comprise one or more fingers 1102 (i.e., extensions, teeth, and/or elongate members) extending around an inner lumen 1114 of the expandable sheath 1100. The one or more fingers 1102 and/or inner lumen 1114 may form a generally circular cross section. The expandable sheath 1100 may comprise a spine 1106 extending along at least a portion of the expandable sheath 1100. In some examples, the one or more fingers 1102 may be inter-connected via connections between the one or more fingers 1102 and the spine 1106. However, the one or more fingers 1102 may not directly couple to and/or contact each other. For example, the one or more fingers 1102 may comprise free ends to allow the one or more fingers 1102 to bend and/or extend as needed to accommodate one or more implants. The one or more fingers 1102 and/or spine 1106 may form an at least partial cylinder around the inner lumen 1114.

In some examples, the one or more fingers 1102 may have a generally curved form and/or may be shape-set in a partial-helix shape. The one or more fingers 1102 may be configured to extend from either side and/or two sides of the spine 1106. In some examples, one or more fingers 1102 extending from a first side of the spine 1106 may be offset from one or more fingers 1102 extending from a second side of the spine 1106. For example, a first finger 1102a may be at least partially offset from a second finger 1102b. The first finger 1102a may extend from a first side of the spine 1106 and the second finger 1102b may extend from a second side of the spine 1106. In some examples, the first finger 1102a may be configured to extend at least partially along the second finger 1102b in a compressed and/or expanded form of the expandable sheath 1100. For example, the first finger 1102a and the second finger 1102b may at least partially overlap with or without contacting each other. Other fingers 1102 may similarly overlap with each other. In some examples, as the expandable sheath 1100 expands, an amount of overlap between the one or more fingers 1102 may decrease as the fingers 1102 straighten and/or are pressed away from each other by an implant passing through the inner lumen 1114.

The expandable sheath 1100 is shown in a compressed or expanded form. In some examples, the one or more fingers 1102 may be configured to overlap in a compressed form and/or to decrease an amount of overlap in an expanded form. Expansion of the expandable sheath 1100 may cause one or more fingers 1102 to at least somewhat straighten.

The spine 1106 may have any suitable size and/or shape. In some examples, the spine 1106 may have a generally rectangular shape and/or may be at least partially curved to facilitate a curvature and/or tubular shape of the expandable sheath 1100. While the spine 1106 is shown comprising a sheet of material, the spine 1106 may be at least partially porous and/or may comprise one or more apertures and/or openings.

The one or more fingers 1102 may have any suitable size and/or shape. In some examples, at least some of the one or more fingers 1102 may have a thin and/or wire-like form. The one or more fingers 1102 may be configured to bend in response to implants passing through the inner lumen 1114. In some examples, the one or more fingers 1102 may be at least partially composed of one or more shape-memory alloys (e.g., Nitinol) and/or may be configured to elastically assume the form shown in Figure 11A and/or 11B upon removal of an implant and/or other external force.

The one or more fingers 1102 may extend from the spine 1106 at different angles. For example, the first finger 1102a and/or the second finger 1102b may extend generally diagonally from the spine 1106 and/or may extend both longitudinally and laterally along the spine 1106. However, while the first finger 1102a may extend at an approximately 45-degree angle form a first end of the spine 1106 (e.g., a distal end of the spine 1106) and/or at an approximately 135-degreen angle from a second end of the spine 1106 (e.g., a proximal end of the spine 1106), the second finger 1102b may extend at an acute and/or approximately 45-degree angle form the second end of the spine 1106 and/or at an approximately 135-degreen angle from the first end of the spine 1106.

The one or more fingers 1102 may not extend fully from both sides of the spine 1106. For example, the first finger 1102a may extend from a first side of the spine 1106 at or near a first end of the spine 1106. The second finger 1102b may extend from a second side of the spine 1106 relatively far from the first end of the spine 1106. For example, a portion of the second side of the spine 1106 at near the first end may not attach to and/or extend into any of the one or more fingers 1102. In some examples, approximately four fingers 1102 may extend from the first side of the spine 1106 at a first portion of the spine 1106 that is at or near the first end of the spine 1106. No fingers 1102 may extend from the second side of the spine 1106 at the first portion. Similarly, the first side of the spine 1106 at or near a second end of the spine 1106 may not attach to and/or extend into any fingers 1102.

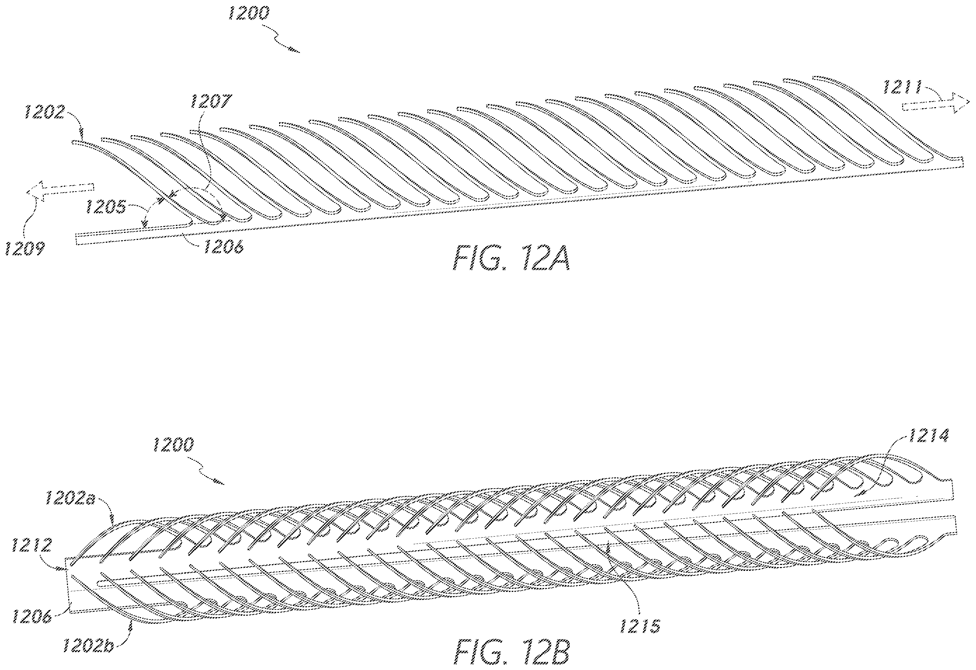

Figures 12A and 12B illustrate an example expandable sheath 1200 comprising in-line and/or wave-shaped (e.g., wavy) fingers 1202 in accordance with one or more examples. The expandable sheath 1200 may have a generally tubular and/or cylindrical shape and/or may comprise one or more fingers 1202 (i.e., extensions, teeth, and/or elongate members) extending around an inner lumen 1214 of the expandable sheath 1200. The one or more fingers 1202 and/or inner lumen 1214 may form a generally circular cross section. The expandable sheath 1200 may comprise a spine 1206 extending along at least a portion of the expandable sheath 1200. The spine 1206 may have an at least partially bisected form and/or may comprise an elongate aperture 1215 extending through and/or bisecting at least a portion of a length of the spine 1206 to facilitate expansion of the expandable sheath 1200. In some examples, the one or more fingers 1202 may be inter-connected via connections between the one or more fingers 1202 and the spine 1206. However, the one or more fingers 1202 may not directly couple to and/or contact each other. For example, the one or more fingers 1202 may comprise free ends to allow the one or more fingers 1202 to bend and/or extend as needed to accommodate one or more implants. The one or more fingers 1202 and/or spine 1206 may form an at least partial cylinder around the inner lumen 1214.

In some examples, the one or more fingers 1202 may have a generally curved form and/or may be shape-set in a semi-circular and/or partial-circular shape. The one or more fingers 1202 may be configured to extend from either side and/or two sides of the spine 1206. In some examples, one or more fingers 1202 extending from a first side of the spine 1206 may be in-line and/or offset from one or more fingers 1202 extending from a second side of the spine 1206. For example, a first finger 1202a may be at least partially in-line with a second finger 1202b. The first finger 1202a may extend from a first side of the spine 1206 and the second finger 1202b may extend from a second side of the spine 1206. In some examples, there may be a gap 1212 between an end of the first finger 1202a and an end of the second finger 1202b. In some examples, as the expandable sheath 1200 expands, the gap 1212 between the fingers 1202 may increase as the fingers 1202 straighten and/or are pressed away from each other by an implant passing through the inner lumen 1214.

The expandable sheath 1200 is shown in a compressed or expanded form. In some examples, expansion of the expandable sheath 1200 may cause one or more fingers 1202 to at least somewhat straighten.

The spine 1206 may have any suitable size and/or shape. In some examples, the spine 1206 may have a generally rectangular shape and/or may be at least partially curved to facilitate a curvature and/or tubular shape of the expandable sheath 1200. The aperture 1215 may be U-shaped and/or may be open-ended at one end portion of the expandable sheath 1200 and/or may be closed at another end portion of the expandable sheath 1200. In some examples, a width of the aperture 1215 may be variable along the length of the expandable sheath 1200 and/or may increase to a maximal width at an open end of the aperture 1215.

The one or more fingers 1202 may have any suitable size and/or shape. In some examples, at least some of the one or more fingers 1202 may have a thin and/or wire-like form. The one or more fingers 1202 may be configured to bend in response to implants passing through the inner lumen 1214. In some examples, the one or more fingers 1202 may be at least partially composed of one or more shape-memory alloys (e.g., Nitinol) and/or may be configured to elastically assume the form shown in Figures 12A and 12B upon removal of an implant and/or other external force. In some examples, the one or more fingers 1202 may have generally wavy forms. For example, the one or more fingers 1202 may extend at a generally 45-degree angle from the spine 1206 and/or may extend generally in line with the spine 1206 at end points of the one or more fingers 1202.

In some examples, the one or more fingers 1202 may have a generally slanted form with respect to the spine 1206, as shown in Figure 12A. For example, a first angle 1205 between a first side of a finger 1202 and the spine 1206 may be less than a second angle 1207 between a second side of the finger 1202 and the spine 1206. Implants may be configured for delivery through the lumen 1214 of the expandable sheath 1200 in a first direction 1209 as opposed to a second direction 1211. Accordingly, movement of an implant through the expandable sheath 1200 in the first direction 1209 may cause the finger 1202 to bend such that the first angle 1205 decreases and/or the second angle 1207 increases. In other words, the finger 1202 may be angled in the direction of movement of the implant to facilitate movement of the implant in the first direction 1209. The first angle 1205 may be an acute angle and/or approximately 45-degrees and/or the second angle 1207 may be obtuse and/or approximately 135-degrees. In other words, in a default form of the sheath 1200, the one or more fingers 1202 may extend at an approximately 45-degree angle with respect to a first end of the spine 1206 (e.g., a distal end from a perspective of an implant entering the inner lumen 1214 of the sheath 1200) and/or may extend at an approximately 135-degree angle with respect to a second end of the spine 1206 (e.g., a proximal end from a perspective of the implant entering the inner lumen 1214 of the sheath 1200).

Figures 13A and 13B illustrate an example expandable sheath 1300 comprising in-line and/or diagonally-extending fingers 1302 in accordance with one or more examples. The expandable sheath 1300 may further comprise a spine 1306 having an at least partially bisected form and/or comprising an elongate aperture 1315 through and/or bisecting at least a portion of a length of the spine 1306 to facilitate expansion of the expandable sheath 1300. In some examples, the one or more fingers 1302 may be inter-connected via connections between the one or more fingers 1302 and the spine 1306. However, the one or more fingers 1302 may not directly couple to and/or contact each other. For example, the one or more fingers 1302 may comprise free ends to allow the one or more fingers 1302 to bend and/or extend as needed to accommodate one or more implants.

The expandable sheath 1300 may have a generally tubular and/or cylindrical shape and/or may comprise one or more fingers 1302 (i.e., extensions, teeth, and/or elongate members) extending around an inner lumen 1314 of the expandable sheath 1300. The one or more fingers 1302 and/or inner lumen 1314 may form a generally circular cross section. The expandable sheath 1300 may comprise a spine 1306 extending along at least a portion of the expandable sheath 1300. The one or more fingers 1302 and/or spine 1306 may form an at least partial cylinder around the inner lumen 1314.

In some examples, the one or more fingers 1302 may have a generally curved form and/or may be shape-set in a semi-circular and/or partial-circular shape. The one or more fingers 1302 may be configured to extend from either side and/or two sides of the spine 1306. In some examples, one or more fingers 1302 extending from a first side of the spine 1306 may be in-line and/or offset from one or more fingers 1302 extending from a second side of the spine 1306. For example, a first finger 1302a may be at least partially in-line with a second finger 1302b. The first finger 1302a may extend from a first side of the spine 1306 and the second finger 1302b may extend from a second side of the spine 1306. In some examples, there may be a gap 1312 between an end of the first finger 1302a and an end of the second finger 1302b. In some examples, as the expandable sheath 1300 expands, the gap 1312 between the fingers 1302 may increase as the fingers 1302 straighten and/or are pressed away from each other by an implant passing through the inner lumen 1314.

The expandable sheath 1300 is shown in a compressed or expanded form. In some examples, expansion of the expandable sheath 1300 may cause one or more fingers 1302 to at least somewhat straighten.

The spine 1306 may have any suitable size and/or shape. In some examples, the spine 1306 may have a generally rectangular shape and/or may be at least partially curved to facilitate a curvature and/or tubular shape of the expandable sheath 1300. The aperture 1315 may be U-shaped and/or may be open-ended at one end portion of the expandable sheath 1300 and/or may be closed at another end portion of the expandable sheath 1300.

The one or more fingers 1302 may have any suitable size and/or shape. In some examples, at least some of the one or more fingers 1302 may have a thin and/or wire-like form. The one or more fingers 1302 may be configured to bend in response to implants passing through the inner lumen 1314. In some examples, the one or more fingers 1302 may be at least partially composed of one or more shape-memory alloys (e.g., Nitinol) and/or may be configured to elastically assume the form shown in Figures 13A and 13B upon removal of an implant and/or other external force.

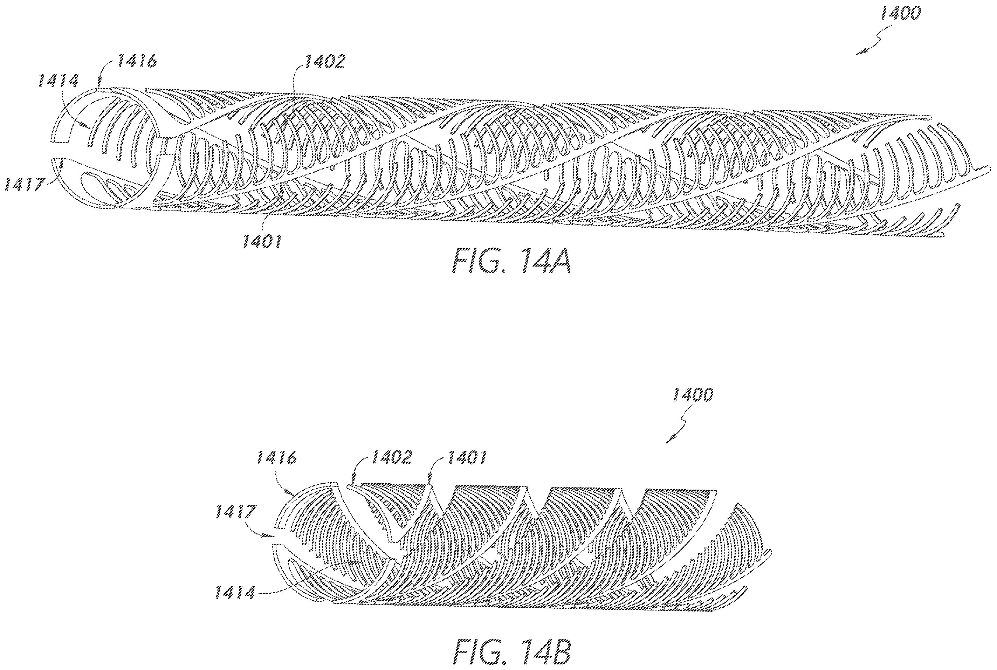

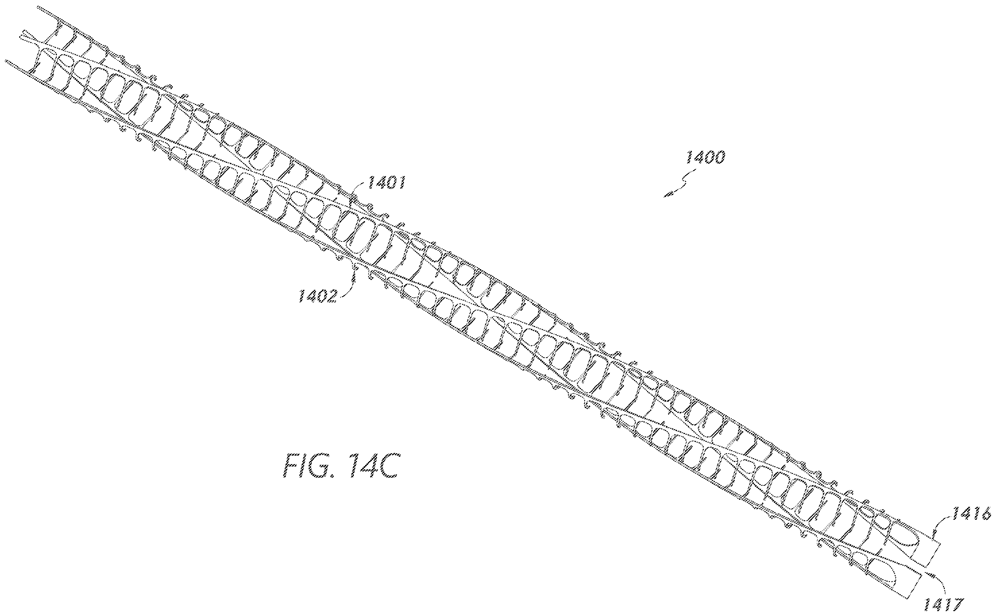

Figures 14A-14C illustrate an expandable sheath 1400 having a generally helical form, in accordance with one or more examples. The expandable sheath 1400 can comprise one or more helical wires 1401 (e.g., fingers and/or spines) situated generally in parallel with each other around a generally tubular and/or cylinder-shaped inner lumen 1414. The expandable sheath 1400 may further comprise one or more rib-like and/or elongate fingers 1402 coupled to and/or extending from the one or more helical wires 1401. While the expandable sheath 1400 is shown comprising four helical wires 1401, the expandable sheath 1400 may comprise any number of helical wires 1401. In some examples, one or more of the fingers 1402 may be inter-connected via connections between the one or more fingers 1402 and the spine one or more helical wires 1401. However, the one or more fingers 1402 may not directly couple to and/or contact each other. For example, the one or more fingers 1402 may comprise free ends to allow the one or more fingers 1402 to bend and/or extend as needed to accommodate one or more implants. Moreover, the one or more helical wires 1401 may similarly comprise free ends to allow the one or more helical wires 1401 to bend and/or extend as needed to accommodate one or more implants.

In some examples, one or more of the helical wires 1401 may be coupled to and/or extend into one or more curved end portions 1416. The one or more curved end portions 1416 may be configured to form a circular and/or partial circular end portion of the expandable sheath 1400. In some examples, multiple curved end portions 1416 may be separated from each other by gaps 1417. Alternatively, the curved end portions 1416 may be coupled to and/or extend into each other and/or may form a continuous circular end portion of the expandable sheath 1400. The one or more helical wires 1401 (e.g., spines), fingers 1402, and/or curved end portions may form an at least partial cylinder around an inner lumen 1414.

In some examples, the one or more helical wires 1401 and/or curved end portions 1416 may not be interconnected and/or may not contact each other. However, the expandable sheath 1400 may comprise a coating (not shown; see, e.g., Figure 6) configured to interconnect the helical wires 1401, fingers 1402, and/or curved end portions 1416. For example, the expandable sheath 1400 may be dip-coated into a material configured to form a uniform layer across at least a portion and/or over the entire expandable sheath 1400. Accordingly, the coating may be configured to hold the separate components of the expandable sheath 1400 together. In some examples, the helical wires 1401 and/or fingers 1402 may provide a skeleton to support the coating. For example, the fingers 1402 may be configured to close gaps between the one or more helical wires 1401 to allow the coating to have a generally tubular form. In some examples, at least some of the one or more helical wires 1401 (e.g., spines) may extend in a first axis and/or direction and/or at least some of the one or more fingers 1402 may extend in a second axis and/or direction (and/or additional axes and/or directions) that is/are orthogonal, perpendicular, and/or at an angle from the first axis and/or direction.

The expandable sheath 1400 may have a generally tubular and/or cylindrical shape. The fingers 1402 (i.e., extensions, teeth, and/or elongate members) may extend around an inner lumen 1414 of the expandable sheath 1400. The one or more fingers 1402 and/or inner lumen 1414 may form a generally circular cross section.

In some examples, the one or more fingers 1402 may have a generally curved form and/or may be shape-set in a curved and/or partial-circular shape. The one or more fingers 1402 may be configured to extend from either side and/or two sides of the spine helical wires 1401. In some examples, fingers 1402 extending from a first helical wire 1401 may be configured to partially cover a gap and/or opening between the first helical wire 1401 and a second helical wire 1401. Similarly, fingers 1402 extending from the second helical wire 1401 may be configured to partially cover a gap and/or opening between the second helical wire 1401 and a third helical wire 1401.

The fingers 1402 and/or helical wires 1401 may be configured to at least partially straighten and/or separate from each other in response to an implant being moved through the inner lumen 1414 of the expandable sheath 1400. The expandable sheath 1400 is shown in a compressed or expanded form.

The one or more fingers 1402 may have any suitable size and/or shape. In some examples, at least some of the one or more fingers 1402 may have a thin and/or wire-like form. The one or more fingers 1402 may be configured to bend in response to implants passing through the inner lumen 1414. In some examples, the one or more fingers 1402 may be at least partially composed of one or more shape-memory alloys (e.g., Nitinol) and/or may be configured to elastically assume the form shown in Figures 14A-14C upon removal of an implant and/or other external force.

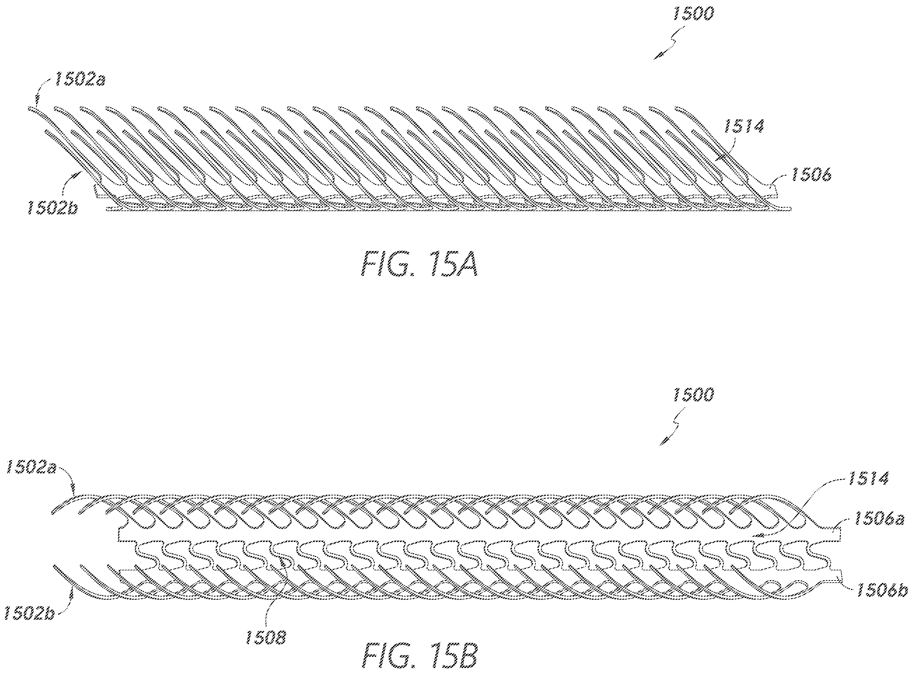

Figures 15A and 15B illustrate an example expandable sheath 1500 comprising one or more in-line and/or wave-shaped fingers 1502 extending from a spine 1506 comprising one or more connecting wires 1508 in accordance with one or more examples. The expandable sheath 1500 may have a generally tubular and/or cylindrical shape and/or may comprise one or more fingers 1502 (i.e., extensions, teeth, and/or elongate members) extending around an inner lumen 1514 of the expandable sheath 1500. The one or more fingers 1502 and/or inner lumen 1514 may form a generally circular cross section. The expandable sheath 1500 may comprise a spine 1506 extending along at least a portion of the expandable sheath 1500. In some examples, the one or more fingers 1502 may be inter-connected via connections between the one or more fingers 1502 and the spine 1506. However, the one or more fingers 1502 may not directly couple to and/or contact each other. For example, the one or more fingers 1502 may comprise free ends to allow the one or more fingers 1502 to bend and/or extend as needed to accommodate one or more implants. The one or more fingers 1502 and/or spine 1506 may form an at least partial cylinder around the inner lumen 1514.

In some examples, the one or more fingers 1502 may have a generally curved form and/or may be shape-set in a semi-circular and/or partial-circular shape. The one or more fingers 1502 may be configured to extend from either side and/or two sides of the spine 1506. In some examples, one or more fingers 1502 extending from a first side of the spine 1506 may be in-line and/or offset from one or more fingers 1502 extending from a second side of the spine 1506. For example, a first finger 1502a may be at least partially in-line with a second finger 1502b extending from an opposing side of the spine 1506. The first finger 1502a may extend from a first side of the spine 1506 and the second finger 1502b may extend from a second side of the spine 1506. In some examples, there may be a gap between an end of the first finger 1502a and an end of the second finger 1502b. In some examples, as the expandable sheath 1500 expands, the gap between the fingers 1502 may increase as the fingers 1502 straighten and/or are pressed away from each other by an implant passing through the inner lumen 1514.

The expandable sheath 1500 is shown in a compressed or expanded form. In some examples, expansion of the expandable sheath 1500 may cause one or more fingers 1502 to at least somewhat straighten.

In some examples, the spine 1506 may comprise multiple components. For example, the spine 1506 may comprise a first spine 1506a connected to a second based portion 1506b via one or more at curved and/or wave-shaped (e.g., wavy) connecting wires 1508. In some examples, the one or more connecting wires 1508 may be configured to at least partially straighten in response to implants moving through the lumen 1514 to facilitate expansion of the expandable sheath 1500.

The one or more fingers 1502 may have any suitable size and/or shape. In some examples, at least some of the one or more fingers 1502 may have a thin and/or wire-like form. The one or more fingers 1502 may be configured to bend in response to implants passing through the inner lumen 1514. In some examples, the one or more fingers 1502 may be at least partially composed of one or more shape-memory alloys (e.g., Nitinol) and/or may be configured to elastically assume the form shown in Figures 15A and 15B upon removal of an implant and/or other external force.

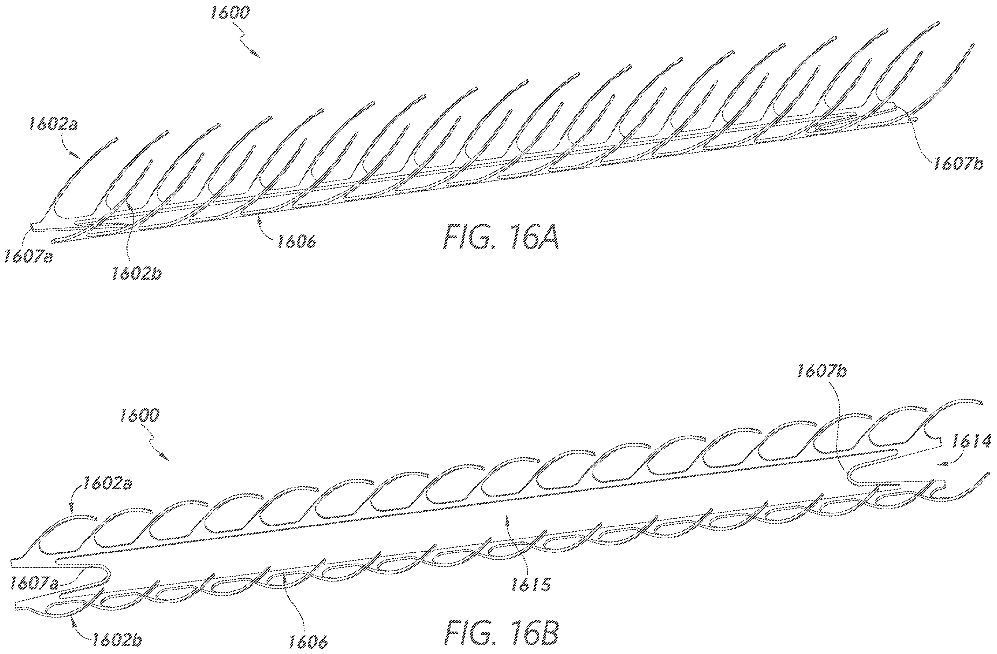

Figures 16A and 16B illustrate an example expandable sheath 1600 comprising one or more in-line and/or curved fingers 1602 extending from either side of a generally hollow spine 1606 in accordance with one or more examples. In some examples, the spine 1606 may have a generally open form in which an elongate aperture 1615 may extend for a portion and/or a majority of a length of the expandable sheath 1600 between a first connector 1607a (e.g., a U-shaped connector) and/or a second connector 1607b. The expandable sheath 1600 may be configured to form a generally cylindrical shape. Fingers 1602 extending from opposing sides of the spine 1606 may be situated in-line with each other and/or may not attach to each other. Accordingly, the expandable sheath 1600 may not form a complete cylinder and/or tube. In some examples, the one or more fingers 1602 may be inter-connected via connections between the one or more fingers 1602 and the spine 1606. However, the one or more fingers 1602 may not directly couple to and/or contact each other. For example, the one or more fingers 1602 may comprise free ends to allow the one or more fingers 1602 to bend and/or extend as needed to accommodate one or more implants.

The expandable sheath 1600 may have a generally tubular and/or cylindrical shape and/or may comprise one or more fingers 1602 (i.e., extensions, teeth, and/or elongate members) extending around an inner lumen 1614 of the expandable sheath 1600. The one or more fingers 1602 and/or inner lumen 1614 may form a generally circular cross section. The expandable sheath 1600 may comprise a spine 1606 extending along at least a portion of the expandable sheath 1600. The one or more fingers 1602 and/or spine 1606 may form an at least partial cylinder around the inner lumen 1614.

In some examples, the one or more fingers 1602 may have a generally curved form and/or may be shape-set in a semi-circular and/or partial-circular shape. The one or more fingers 1602 may be configured to extend from either side and/or two sides of the spine 1606. In some examples, one or more fingers 1602 extending from a first side of the spine 1606 may be in-line and/or offset from one or more fingers 1602 extending from a second side of the spine 1606. For example, a first finger 1602a may be at least partially in-line with a second finger 1602b. The first finger 1602a may extend from a first side of the spine 1606 and the second finger 1602b may extend from a second side of the spine 1606. In some examples, there may be a gap between an end of the first finger 1602a and an end of the second finger 1602b. In some examples, as the expandable sheath 1600 expands, the gap between the fingers 1602 may increase as the fingers 1602 straighten and/or are pressed away from each other by an implant passing through the inner lumen 1614.

In some examples, the fingers 1602 may extend diagonally (e.g., at an acute and/or approximately 45-degree angle) with respect to the spine 1606. The one or more fingers 1602 may be angled in a direction of insertion of one or more implants through the expandable sheath 1600. In this way, the orientation of the fingers 1602 may facilitate movement of one or more implants through the expandable sheath 1600.

The expandable sheath 1600 is shown in a compressed or expanded form. In some examples, expansion of the expandable sheath 1600 may cause one or more fingers 1602 to at least somewhat straighten and/or separate from each other. For example, in response to an implant passing between the first finger 1602a and the second finger 1602b, a gap and/or distance between at least end points of the first finger 1602a and second finger 1602b may increase.

The one or more fingers 1602 may have any suitable size and/or shape. In some examples, at least some of the one or more fingers 1602 may have a thin and/or wire-like form. The one or more fingers 1602 may be configured to bend in response to implants passing through the inner lumen 1614. In some examples, the one or more fingers 1602 may be at least partially composed of one or more shape-memory alloys (e.g., Nitinol) and/or may be configured to elastically assume the form shown in Figures 16A and 16B upon removal of an implant and/or other external force.

In some examples, the spine 1606 may comprise one or more connectors 1607 configured to extend across the aperture 1615 to form a bridge between fingers 1602 on opposing sides of the spine 1606. The expandable sheath 1600 may comprise a first connector 1607a at a first end portion of the expandable sheath 1600 and/or may comprise a second connector 1607b at a second end portion of the expandable sheath 1600. The connectors 1607 may have generally curved and/or U-shaped forms. In some examples, the connectors 1607 may be configured to at least partially straighten in response to implants passing through the lumen 1614 of the expandable sheath 1600 to increase a diameter and/or width of the expandable sheath 1600.

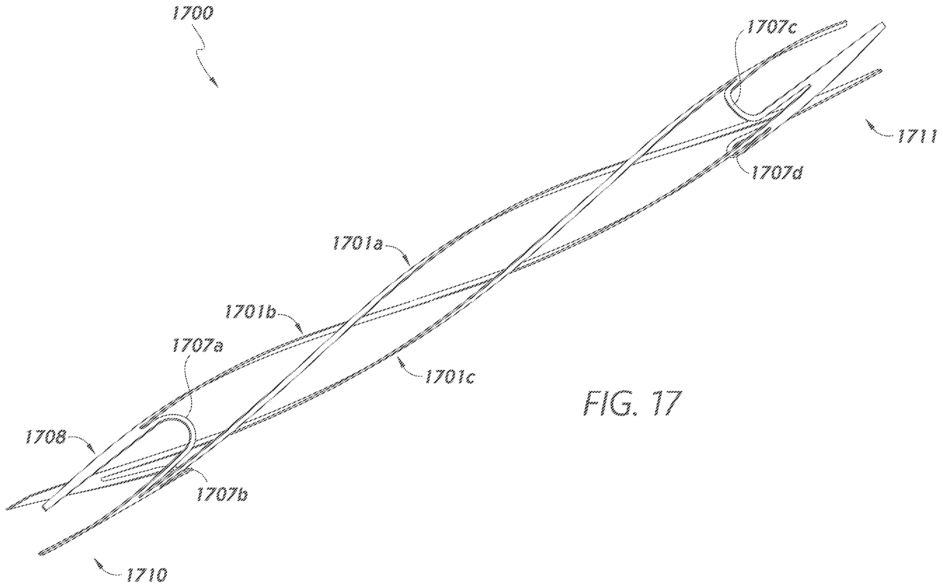

Figure 17 illustrates an expandable sheath 1700 having a generally helical form, in accordance with one or more examples. The expandable sheath 1700 can comprise one or more helical wires 1701 (e.g., fingers and/or spines) situated generally in parallel with each other and/or extending in a generally helical form around a generally tubular and/or cylindrical inner lumen. The expandable sheath 1700 may further comprise one or more elongate fingers coupled to and/or extending from the one or more helical wires 1701. For example, one or more fingers may extend generally perpendicularly from the one or more helical wires 1701 and/or may extend between multiple helical wires 1701 of the one or more helical wires 1701. While the expandable sheath 1700 is shown comprising four helical wires 1701, the expandable sheath 1700 may comprise any number of helical wires 1701. The one or more helical wires 1701 (e.g., spines) and/or fingers may form an at least partial cylinder around an inner lumen.

In some examples, the expandable sheath 1700 may comprise a coating (not shown; see, e.g., Figure 6) configured to interconnect the helical wires 1701 and/or one or more fingers and/or wires interconnecting the helical wires 1701. For example, the expandable sheath 1700 may be dip-coated into a material configured to form a uniform layer across at least a portion and/or over the entire expandable sheath 1700. Accordingly, the coating may be configured to hold the separate components of the expandable sheath 1700 together. In some examples, the helical wires 1701 and/or fingers may provide a skeleton to support the coating. For example, the fingers may be configured to close gaps between the one or more helical wires 1701 to allow the coating to have a generally tubular form.

The expandable sheath 1700 may have a generally tubular and/or cylindrical shape. The helical wires 1701 (i.e., elongate members) may extend around an inner lumen of the expandable sheath 1700. The one or more helical wires 1701 and/or inner lumen may form a generally circular cross section.

In some examples, the one or more helical wires 1701 may have a generally curved form and/or may be shape-set in a curved and/or partial-circular shape. In some examples, fingers extending from a first helical wire 1701 may be configured to partially cover a gap and/or opening between the first helical wire 1701 and a second helical wire 1701. Similarly, fingers extending from the second helical wire 1701 may be configured to partially cover a gap and/or opening between the second helical wire 1701 and a third helical wire 1701.

The helical wires 1701 may be configured to at least partially straighten and/or separate from each other in response to an implant being moved through the inner lumen of the expandable sheath 1700. In some examples, at least some of the one or more helical wires 1701 may have a thin and/or wire-like form. The one or more helical wires 1701 may be configured to bend in response to implants passing through the inner lumen. In some examples, the one or more helical wires 1701 may be at least partially composed of one or more shape-memory alloys (e.g., Nitinol) and/or may be configured to elastically assume the form shown in Figure 17 upon removal of an implant and/or other external force.

In some examples, one or more of the helical wires 1701 may be coupled to other helical wires 1701 via one or more connectors 1707 (e.g., U-shaped connectors). A connector 1707 may comprise one or more lines (e.g., wires) extending between two helical wires 1701. In some examples, one or more connectors 1707 may have generally curved forms. For example, a connector 1707 may be U-shaped in a compressed form of the expandable sheath 1700 and/or may be configured to at least partially straighten in response to an implant being passed through the one or more helical wires 1701.

The connectors 1707 may couple to and/or extend into the one or more helical wires 1701 at one or more end portion 1708 of the expandable sheath 1700. An end portion 1708 may comprise an elongate and/or generally straight extension of a helical wire 1701 and/or connector 1707. In some examples, the one or more helical wires 1701, connectors 1707, and/or end portions 1708 may be at least partially composed of common materials (e.g., Nitinol and/or other shape-memory materials) and/or different materials. The one or more helical wires 1701 and/or connectors 1707 may have approximately equal widths. In some examples, the end portions 1708 may have approximately 2x the width of the one or more helical wires 1701 and/or connectors 1707.

In some examples, the one or more helical wires 1701 may be inter-connected via connections between the one or more helical wires 1701 and the one or more end portion 1708. However, the one or more helical wires 1701 may not directly couple to and/or contact each other.

In some examples, the expandable sheath 1700 may comprise any number of connectors 1707 and/or helical wires 1701. For example, the expandable sheath 1700 may comprise, at a first end 1710 of the expandable sheath 1700, a first connector 1707a connecting a first helical wire 1701a to a second helical wire 1701b and/or may comprise a second connector 1707b connecting the first helical wire 1701a to a third helical wire 1701c. The expandable sheath 1700 may not comprise a connector 1707 connecting the second helical wire 1701b to the third helical wire 1701c at the first end 1710. Similarly, the expandable sheath 1700 may comprise, at a second end 1711 of the expandable sheath 1700, a third connector 1707c connecting the second helical wire 1701b to the third helical wire 1701c and/or may comprise a fourth connector 1707d connecting the first helical wire 1701a to the second helical wire 1701b. The expandable sheath 1700 may not comprise a connector 1707 connecting the first helical wire 1701a to the third helical wire 1701c at the second end 1711.

While the helical wires 1701 are shown connecting only at the first end 1710 and/or second end 1711, the expandable sheath 1700 may comprise additional connection points between the helical wires 1701. For example, the expandable sheath 1700 may comprise additional wires interconnecting the various helical wires 1701 along the length of the expandable sheath 1700. The additional wires can extend generally perpendicularly, laterally, and/or diagonally with respect to the helical wires 1701. In some examples, the helical wires 1701 and/or any additional wires interconnecting the helical wires 1701 can provide support for a coating which may be applied to the expandable sheath 1700.

Some implementations of the present disclosure relate to a delivery sheath comprising inter-connected fingers extending around a cylindrical inner lumen and forming one or more gaps. The inter-connected fingers are configured to assume a default form in which the inner lumen has a first width, bend in response to an implant passing through the inner lumen to cause at least a portion of the inner lumen to increase beyond the first width, and elastically return to the default form following removal of the implant from the inner lumen.

The delivery sheath may further comprise an expandable coating enclosing at least a portion of the one or more gaps.

In some examples, the delivery sheath further comprises a spine extending along at least a portion of the inner lumen. The inter-connected fingers may form rib-like curved extensions from the spine.

A first portion of the inter-connected fingers may extend from a first side of the spine and/or a second portion of the inter-connected fingers may extend from a second side of the spine. In some examples, the first portion of the inter-connected fingers may be generally in-line with the second portion of the inter-connected fingers.

In some examples, the first portion of the inter-connected fingers are at least partially offset from the second portion of the inter-connected fingers. The first portion of the inter-connected fingers may at least partially overlap with the second portion of the inter-connected fingers.

The inter-connected fingers may extend generally perpendicularly from the spine.

In some examples, the first portion of inter-connected fingers extend at an approximately 45-degree angle from a first end of the spine. The inner lumen may be configured to receive an implant moving towards the first end of the spine.

The second portion of inter-connected fingers may extend at an approximately 45-degree angle from the first end of the spine. In some examples, the second portion of inter-connected fingers extend at an approximately 45-degree angle from a second end of the spine.

In some examples, the spine comprises a solid, curved, and/or generally rectangular sheet of material. The spine may comprise a partially bisected, curved, and/or generally rectangular sheet of material.

The spine may comprise a generally rectangular frame having an elongate aperture.

In some examples, the delivery sheath further comprises multiple helical spines extending around the inner lumen. The inter-connected fingers may form rib-like curved extensions from the multiple helical spines.

The inter-connected fingers may extend in a helical form around the inner lumen. In some examples, the inter-connected fingers are at least partially composed of one or more shape-memory alloys.

In accordance with one or more implementations of the present disclosure, a delivery sheath comprises one or more spines and one or more fingers extending from the one or more spines. The one or more spines and one or more fingers form an at least partial cylinder around an inner lumen. The one or more fingers are configured to extend to increase a width of the inner lumen in response to an implant being passed through the inner lumen.

At least some of the one or more spines may extend along a first axis and at least some of the one or more fingers extend generally perpendicularly from the first axis.

### Additional Examples

Depending on the example, certain acts, events, or functions of any of the processes or algorithms described herein can be performed in a different sequence, may be added, merged, or left out altogether. Thus, in certain examples, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain examples include, while other examples do not include, certain features, elements and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single example, Figure, or description thereof for the purpose of streamlining the disclosure and aiding in the understanding of one or more of the various aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A delivery sheath (1200) comprising:
inter-connected fingers (1202) extending around a cylindrical inner lumen (1214) and forming one or more gaps (1212), the inter-connected fingers (1202) configured to:
assume a default form in which the inner lumen (1214) has a first width;
bend in response to an implant (403) passing through the inner lumen (1214) to cause at least a portion of the inner lumen (1214) to increase beyond the first width; and
elastically return to the default form following removal of the implant (403) from the inner lumen (1214); and
a spine (1206) extending along at least a portion of the inner lumen (1214), wherein the inter-connected fingers (1202) form rib-like curved extensions from the spine (1206); and
wherein the spine (1206) comprises an elongate aperture (1215).

2. The delivery sheath (1200) of claim 1, further comprising an expandable coating enclosing at least a portion of the one or more gaps (1212).

3. The delivery sheath (1200) of claim 1, wherein a first set (1202a) of the inter-connected fingers (1202) extend from a first side of the spine (1206) and a second set (1202b) of the inter-connected fingers (1202) extend from a second side of the spine (1206).

4. The delivery sheath (1200) of claim 3, wherein the first set (1202a) of the inter-connected fingers (1202) are generally in-line with the second set (1202b) of the inter-connected fingers (1202).

5. The delivery sheath (1200) of claim 3 or claim 4, wherein the first set (1202a) of the inter-connected fingers (1202) are at least partially offset from the second set (1202b) of the inter-connected fingers (1202).

6. The delivery sheath (1200) of claim 5, wherein the first set (1202a) of the inter-connected fingers (1202) at least partially overlap with the second set (1202b) of the inter-connected fingers (1202).

7. The delivery sheath (1200) of any of claims 3-6, wherein the inter-connected fingers (1202) extend generally perpendicularly from the spine (1206).

8. The delivery sheath (1200) of any of claims 3-7, wherein the first set (1202a) of inter-connected fingers (1202) extend at an acute angle from a first end of the spine (1206).

9. The delivery sheath (1200) of claim 8, wherein the inner lumen (1214) is configured to receive an implant (403) moving towards the first end of the spine (1206).

10. The delivery sheath (1200) of claim 8 or claim 9, wherein the second set (1202b) of inter-connected fingers (1202) extend at an acute angle from the first end of the spine (1206).

11. The delivery sheath (1200) of any of claims 8-10, wherein the second set (1202b) of inter-connected fingers (1202) extend at an acute angle from a second end of the spine (1206).

12. The delivery sheath (1200) of any of claims 1-11, wherein the spine (1206) comprises a partially bisected and curved sheet of material.

13. The delivery sheath (1200) of any of claims 1-12, wherein the spine (1206) has a generally rectangular shape.

14. The delivery sheath (1200) of any of claims 1-13, further comprising multiple helical spines (1401) extending around the inner lumen (1214), wherein the inter-connected fingers (1202) form rib-like curved extensions from the multiple helical spines (1401).

15. The delivery sheath (1200) of any of claims 1-14, wherein the inter-connected fingers (1202) extend in a helical form around the inner lumen (1214).

## Patentansprüche

1. Zuführhülle (1200), umfassend:
miteinander verbundene Finger (1202), die sich um ein zylindrisches Innenlumen (1214) erstrecken und einen oder mehrere Spalte (1212) bilden, wobei die miteinander verbundenen Finger (1202) ausgestaltet sind zum:
Annehmen einer Standardform, in der das Innenlumen (1214) eine erste Breite hat;
Biegen in Reaktion darauf, dass ein Implantat (403) das Innenlumen (1214) passiert, um zu bewirken, dass mindestens ein Abschnitt des Innenlumens (1214) über die erste Breite hinaus zunimmt; und
elastisches Zurückkehren zu der Standardform nach Entfernung des Implantats (403) von dem Innenlumen (1214); und
ein Rückgrat (1206), das sich entlang mindestens eines Abschnitts des Innenlumens (1214) erstreckt, wobei die miteinander verbundenen Finger (1202) rippenartige gekrümmte Fortsätze von dem Rückgrat (1206) bilden; und
wobei das Rückgrat (1206) eine längliche Öffnung (1215) umfasst.

2. Zuführhülle (1200) nach Anspruch 1, des Weiteren umfassend eine expandierbare Beschichtung, die mindestens einen Abschnitt des einen Spalts oder der mehreren Spalte (1212) umschließt.

3. Zuführhülle (1200) nach Anspruch 1, wobei ein erster Satz (1202a) der miteinander verbundenen Finger (1202) sich von einer ersten Seite des Rückgrats (1206) erstreckt, und ein zweiter Satz (1202b) der miteinander verbundenen Finger (1202) sich von einer zweiten Seite des Rückgrats (1206) erstreckt.

4. Zuführhülle (1200) nach Anspruch 3, wobei der erste Satz (1202a) der miteinander verbundenen Finger (1202) allgemein in einer Linie mit dem zweiten Satz (1202b) der miteinander verbundenen Finger (1202) ist.

5. Zuführhülle (1200) nach Anspruch 3 oder Anspruch 4, wobei der erste Satz (1202a) der miteinander verbundenen Finger (1202) mindestens teilweise versetzt zu dem zweiten Satz (1202b) der miteinander verbundenen Finger (1202) ist.

6. Zuführhülle (1200) nach Anspruch 5, wobei der erste Satz (1202a) der miteinander verbundenen Finger (1202) mindestens teilweise mit dem zweiten Satz (1202b) der miteinander verbundenen Finger (1202) überlappt.

7. Zuführhülle (1200) nach einem der Ansprüche 3 bis 6, wobei die miteinander verbundenen Finger (1202) sich allgemein rechtwinklig von dem Rückgrat (1206) erstrecken.

8. Zuführhülle (1200) nach einem der Ansprüche 3 bis 7, wobei der erste Satz (1202a) der miteinander verbundenen Finger (1202) sich in einem spitzen Winkel von einem ersten Ende des Rückgrats (1206) erstreckt.

9. Zuführhülle (1200) nach Anspruch 8, wobei das Innenlumen (1214) ausgestaltet ist, um ein Implantat (403) aufzunehmen, das sich in Richtung des ersten Endes des Rückgrats (1206) bewegt.

10. Zuführhülle (1200) nach Anspruch 8 oder Anspruch 9, wobei der zweite Satz (1202b) der miteinander verbundenen Finger (1202) sich in einem spitzen Winkel von dem ersten Ende des Rückgrats (1206) erstreckt.

11. Zuführhülle (1200) nach einem der Ansprüche 8 bis 10, wobei der zweite Satz (1202b) der miteinander verbundenen Finger (1202) sich in einem spitzen Winkel von einem zweiten Ende des Rückgrats (1206) erstreckt.

12. Zuführhülle (1200) nach einem der Ansprüche 1 bis 11, wobei das Rückgrat (1206) eine teilweise zweigeteilte und gekrümmte Lage aus Material umfasst.

13. Zuführhülle (1200) nach einem der Ansprüche 1 bis 12, wobei das Rückgrat (1206) eine allgemein rechteckige Formgebung hat.

14. Zuführhülle (1200) nach einem der Ansprüche 1 bis 13, des Weiteren umfassend mehrere helixförmige Rückgrate (1401), die sich um das Innenlumen (1214) herum erstrecken, wobei die miteinander verbundenen Finger (1202) rippenartige gekrümmte Fortsätze von den mehreren helixförmigen Rückgraten (1401) bilden.

15. Zuführhülle (1200) nach einem der Ansprüche 1 bis 14, wobei die miteinander verbundenen Finger (1202) sich in einer Helixform um das Innenlumen (1214) herum erstrecken.

## Revendications

1. Gaine de pose (1200), comprenant :
des doigts interconnectés (1202) s'étendant autour d'une lumière interne (1214) cylindrique et formant un ou plusieurs interstices (1212), les doigts interconnectés (1202) étant conçus pour :
adopter une forme par défaut dans laquelle la lumière interne (1214) présente une première largeur ;
se fléchir en réponse à un implant (403) traversant la lumière interne (1214) afin d'amener au moins une partie de la lumière interne (1214) à augmenter au-delà de la première largeur ; et
revenir élastiquement à la forme par défaut suite après le retrait de l'implant (403) de la lumière interne (1214) ; et
une structure (1206) s'étendant le long d'au moins une partie de la lumière interne (1214), les doigts interconnectés (1202) formant des extensions incurvées de type nervure à partir de la structure (1206) ; et
la structure (1206) comprenant une ouverture allongée (1215).

2. Gaine de pose (1200) selon la revendication 1, comprenant en outre un revêtement extensible entourant au moins une partie dudit un ou desdits plusieurs interstices (1212).

3. Gaine de pose (1200) selon la revendication 1, un premier ensemble (1202a) des doigts interconnectés (1202) s'étendant à partir d'un premier côté de la structure (1206) et un second ensemble (1202b) des doigts interconnectés (1202) s'étendant à partir d'un second côté de la structure (1206).

4. Gaine de pose (1200) selon la revendication 3, le premier ensemble (1202a) des doigts interconnectés (1202) étant généralement aligné sur le second ensemble (1202b) des doigts interconnectés (1202).

5. Gaine de pose (1200) selon la revendication 3 ou la revendication 4, le premier ensemble (1202a) des doigts interconnectés (1202) étant au moins partiellement décalé par rapport au second ensemble (1202b) des doigts interconnectés (1202).

6. Gaine de pose (1200) selon la revendication 5, le premier ensemble (1202a) des doigts interconnectés (1202) chevauchant au moins partiellement le second ensemble (1202b) des doigts interconnectés (1202).

7. Gaine de pose (1200) selon l'une quelconque des revendications 3 à 6, les doigts interconnectés (1202) s'étendant généralement perpendiculairement à partir de la structure (1206).

8. Gaine de pose (1200) selon l'une quelconque des revendications 3 à 7, le premier ensemble (1202a) de doigts interconnectés (1202) s'étendant selon un angle aigu à partir d'une première extrémité de la structure (1206).

9. Gaine de pose (1200) selon la revendication 8, la lumière interne (1214) étant conçue pour recevoir un implant (403) se déplaçant vers la première extrémité de la structure (1206).

10. Gaine de pose (1200) selon la revendication 8 ou selon la revendication 9, le second ensemble (1202b) de doigts interconnectés (1202) s'étendant selon un angle aigu à partir de la première extrémité de la structure (1206).

11. Gaine de pose (1200) selon l'une quelconque des revendications 8 à 10, le second ensemble (1202b) de doigts interconnectés (1202) s'étendant selon un angle aigu à partir d'une seconde extrémité de la structure (1206).

12. Gaine de pose (1200) selon l'une quelconque des revendications 1 à 11, la structure (1206) comprenant une feuille de matériau partiellement bissectée et incurvée.

13. Gaine de pose (1200) selon l'une quelconque des revendications 1 à 12, la structure (1206) présentant une forme généralement rectangulaire.

14. Gaine de pose (1200) selon l'une quelconque des revendications 1 à 13, comprenant en outre de multiples structures hélicoïdales (1401) s'étendant autour de la lumière interne (1214), les doigts interconnectés (1202) formant des extensions incurvées de type nervure à partir des multiples structures hélicoïdales (1401).

15. Gaine de pose (1200) selon l'une quelconque des revendications 1 à 14, les doigts interconnectés (1202) s'étendant sous une forme hélicoïdale autour de la lumière interne (1214).
